# EUROPEAN PATENT SPECIFICATION

(11) **EP 3 076 885 B1**
(45) Date of publication and mention of the grant of the patent: **09.10.2019**
(21) Application number: 14815080.8
(22) Date of filing: 04.12.2014
(51) Int. Cl.: A61B 17/34, A61B 90/50, A61B 17/29

(54) **ACCESS DEVICE AND ASSEMBLY COMPRISING SUCH DEVICE**
ZUGANGSVORRICHTUNG UND ANORDNUNG MIT SOLCH EINER VORRICHTUNG
DISPOSITIF D'ACCÈS ET ENSEMBLE COMPRENANT CE DISPOSITIF

(30) Priority: 04.12.2013 WO PCT/NL2013/050873
(43) Date of publication of application: 12.10.2016
(73) Proprietor: Fortimedix Surgical B.V., 6361 HK Nuth (NL)
(72) Inventor: VERBEEK, Maurice Theodoor Ivonne, NL-6166 JP Geleen (NL); VERBEEK, Marcel Antonius Elisabeth, NL-6369 SR Heerlen (NL); PLEIJERS, Simon Jozef Arnold, NL-6343 AS Klimmen (NL); NIJSTEN, Frank Johannes Hendrikus, NL-6049 KN Herten (NL); THISSEN, Mattheus Hendrik Louis, NL-6071 NC Swalmen (NL)
(74) Representative: Nederlandsch Octrooibureau
(86) International application number: PCT/NL2014/050832
(87) International publication number: WO 2015/084174

(56) References cited:
- EP-A1- 2 269 516
- EP-A1- 2 430 991
- EP-A2- 2 226 024
- WO-A1-2010/096580
- WO-A2-2010/042915
- US-A1- 2012 253 131
- US-A1- 2013 317 522

## Description

### FIELD OF THE INVENTION

The invention relates to an assembly comprising at least one invasive instrument and an access device that is adapted for introducing the at least one invasive instrument into an object via an access port of the object. The invention further relates to the access device.

The assembly and the access device according to the invention can be used in both medical and non-medical applications. Examples of the latter include inspection and/or repair of mechanical and/or electronic hardware at locations that are difficult to reach. Therefore, the object mentioned above can for example be a human or animal body but also mechanical and/or electronic hardware. Hence, terms used in the following description such as endoscopic application or invasive instrument, must be interpreted in a broad manner.

### BACKGROUND OF THE INVENTION

Transformation of surgical interventions that require large incisions for exposing a target area into minimal invasive surgical interventions, i.e. requiring only natural orifices or small incisions for establishing access to the target area, is a well-known and ongoing process. In performing minimal invasive surgical interventions, an operator such as a physician, requires an access device that is arranged for introducing and guiding invasive instruments into the human or animal body via an access port of that body. In order to reduce scar tissue formation and pain to a human or animal patient, the access port is preferably provided by a single small incision in the skin and underlying tissue. In that respect the possibility to use a natural orifice of the body would even be better. Furthermore, the access device preferably enables the operator to control at least one degree of freedom that the invasive instruments offer. In this way, the operator can perform required actions at the target area in the human or animal body in an ergonomic and accurate manner with a reduced risk of clashing of the instruments used.

Surgical invasive instruments and endoscopes through which these instruments are guided towards the target area are well-known in the art. Both the invasive instruments and endoscopes can comprise a steerable tube that enhances its navigation and steering capabilities. Such a steerable tube preferably comprises a proximal end part including at least one flexible zone, a distal end part including at least one flexible zone, and a rigid intermediate part, wherein the steerable tube further comprises a steering arrangement that is adapted for translating a deflection of at least a part of the proximal end part relative to the rigid intermediate part into a related deflection of at least a part of the distal end part.

Furthermore, the steerable tube preferably comprises a number of co-axially arranged cylindrical elements including an outer element, an inner element and one or more intermediate elements depending on the number of flexible zones in the proximal and distal end parts of the tube and the desired implementation of the steering members of the steering arrangement, i.e. all steering members can be arranged in a single intermediate element or the steering members are divided in different sets and each set of steering members is arranged in a different intermediate member. The steering arrangement may comprise conventional steering cables with sub 1mm diameters as steering members, wherein the steering cables are arranged between related flexible zones at the proximal and distal end parts of the tube. However, as steering cables have many well-known disadvantages, it is preferred to avoid them and to implement the steering members by one or more sets of longitudinal elements that form integral parts of the one or more intermediate elements. Each of the intermediate elements can be fabricated either by using a suitable material addition technique, such as injection moulding or plating, or by a suitable material removal technique, such as laser cutting, photochemical etching, deep pressing, conventional chipping techniques such as drilling or milling or high-pressure water jet cutting systems. Of the aforementioned material removal techniques, laser cutting is very advantageous as it allows a very accurate and clean removal of material under reasonable economic conditions. Further details regarding the design and fabrication of the abovementioned steerable tube and the steering arrangement thereof have been described for example in WO 2009/112060 A1, WO 2009/127236 A1, US 13/160,949, and US 13/548,935 of the applicant.

Steerable invasive instruments typically comprise a handle that is arranged at the proximal end part of the steerable tube for steering the tube and/or for manipulating a tool that is arranged at the distal end part of the steerable tube. Such a tool can for example be a camera, a manual manipulator, e.g. a pair of scissors, forceps, or manipulators using an energy source, e.g. an electrical, ultrasonic or optical energy source.

In this application, the terms "proximal" and "distal" are defined with respect to an operator, e.g. a physician that operates the instrument or endoscope. For example, a proximal end part is to be construed as a part that is located near the physician and a distal end part as a part located at a distance from the physician.

An common single port access device that is used in minimal invasive surgical interventions typically enables to introduce and guide two or more steerable invasive instruments into a human or animal body via a single incision or a natural orifice. For the removal of tissue, typically two steerable instruments are required, wherein a first instrument for example comprising a pair of grasping forceps is used for taking hold of the tissue to be removed and a second instrument for example comprising an electromechanical cutting device is used for dissecting the tissue. As the two steerable instruments in this non-limiting example are introduced via a common guiding arrangement of the access device, wherein the guiding arrangement typically comprises a guide tube including separate lumen one for each instrument, the steerable tubes of the instruments are closely spaced with respect to each other in a radial direction of the guide tube. As the two instruments thus enter the body very close to one another, it is required to either provide spreading means such as a controllable wedge, an inflatable-deflatable balloon or spreader arms near the distal end parts of the steerable instruments or to use pre-bent or steerable guiding tubes and/or endoscopes in order to achieve sufficient triangulation of the distal end parts of the instruments, i.e. achieving a geometric orientation of the distal end parts of the instruments that provides the sufficient space for maneuvering and positioning the tools at the operation site. Access devices well-known in the art as have been described in for example US 2008/0188868 A1 and WO 2011/014711 A1, apply at least one of the abovementioned measures to achieve sufficient triangulation.

A disadvantage of known access devices like the ones that are described in the documents mentioned above, is that the construction of these access devices is more complicated and therefore more costly because of the implementation of a spreading means, wherein the spreading means is arranged at the distal side of the access device which in use of the access device is located inside an object and wherein the spreading means is operated by a control means that is arranged at the proximal side of the access device which in use of the access device is located outside the object. As mentioned above, the object in the meaning of the present invention is not limited to a human or animal body but can also be a piece of mechanical and/or electronic hardware.

EP 2 430 991 A1 describes an example of a medical manipulator including an arm formed in a tubular shape having a bending portion capable of bending, an insertion portion that is formed having channels, with the base end of the arm being connected to the distal end of the insertion portion so that lumens of the arm and the channels are in communication, an operation portion for operating the bending portion, and transmission members that connect the bending portion and the operation portion, wherein the operation portion includes an operation main body that supports the transmission members so as to being capable of freely advancing and retracting in the direction in which each of the transmission members extend, a lever that has a tubular portion that allows insertion of the treatment tool, with one end side of the tubular portion being coupled to each of the transmission members via links, and the other end of the tubular portion capable of swinging about a rotation center that is established by the relative position with respect to the operation main body being fixed, and a guide member that is fixed to the operation main body and coupled to the lever, and that guides the lever so that the turning angle about the rotation center at the other end of the lever is smaller than the turning angle about the rotation center at the one end.

EP 2 226 024 A2 describes another example of a surgical access device comprising a retractor having a working channel extending therethrough for forming a pathway through tissue into a body cavity, and a housing having a plurality of sealing ports configured to receive an instrument therethrough and into the work channel. The housing is configured to releasably mate to the retractor in only one predetermined rotational orientation.

EP 2 269 516 A1 describes another example of a surgical apparatus for transanal endoscopic microsurgery capable of adjusting an operation area by adding a rotation function to a barrel of the surgical apparatus without separation of the surgical apparatus from a fixing frame during an operation.

However, none of the prior art references allow engagement of a steerable invasive instrument which distinct from the access device such that the steerable instrument can be deflected and moved in an axial direction.

### SUMMARY OF THE INVENTION

It is an object of the invention to provide an assembly comprising at least one steerable invasive instrument and an access device, wherein the assembly preempts or at least reduces the disadvantage of the known access devices mentioned above.

It is also an object of the invention to provide an access device having a less complicated construction for establishing triangulation of the distal end part of at least one steerable invasive instrument that can be received and guided by the access device.

The invention provides an access device according to claim 1. Further embodiments of the invention are provided in the dependent claims. Claim 12 is directed to an assembly comprising the access device according to the invention.

In an embodiment of the assembly according to the invention, the assembly comprises at least one invasive instrument and an access device that is adapted for introducing the at least one invasive instrument into an object via an access port of the object, wherein the invasive instrument is at least one of:
a rigid invasive instrument comprising a rigid elongated tubular body; and
a steerable invasive instrument comprising an elongated tubular body having a proximal end part comprising at least one flexible zone, a distal end part comprising at least one flexible zone, and a rigid intermediate part, wherein the steerable invasive instrument further comprises a steering arrangement that is adapted for translating a deflection of at least a part of the proximal end part relative to the rigid intermediate part into a related deflection of at least a part of the distal end part;
wherein the access device comprises
a guiding arrangement that is adapted for receiving and guiding the invasive instrument into the object in at least one guiding direction; and
a deflection setting arrangement that is adapted for setting at least one angle between at least a part of the proximal end part and the rigid intermediate part of a steerable invasive instrument.

As mentioned above, the object in the meaning of the present invention is not limited to a human or animal body but can also be a piece of mechanical and/or electronic hardware. The access port of a human or animal body is preferably provided by a single small incision in the skin and underlying tissue. However, depending on the intervention, the access port could also be provided by a natural orifice of the body.

As described above, the steerable tube of a steerable invasive instrument preferably comprises a number of co-axially arranged cylindrical elements including an outer element, an inner element and one or more intermediate elements depending on the number of flexible zones in the proximal and distal end parts of the tube and the desired implementation of the steering members of the steering arrangement, i.e. all steering members can be arranged in a single intermediate cylindrical element or the steering members are divided in different sets and each set of steering members is arranged in a different intermediate cylindrical element. Hence, the elongated tubular body of the steerable invasive instrument according to the invention preferably comprises a number of cylindrical elements as described above. Although the steering arrangement of the steerable invasive instrument may comprise conventional steering cables, it preferably comprises one or more sets of longitudinal elements that form integral parts of the intermediate element. Preferably the longitudinal elements are remaining parts of the wall of the intermediate element after longitudinal slits have been made in the wall of the intermediate element. The longitudinal slits are preferably made by laser cutting but the skilled person will have knowledge of other suitable material removal techniques. It is also possible to form the longitudinal elements during the fabrication of the intermediate element itself. This can be done by suitable material addition techniques such as injection moulding or plating.

The access device according to the invention comprises a guiding arrangement for receiving and guiding for example two steerable invasive instruments into an object. In other embodiments according to the invention, the guiding arrangement can receive and guide a rigid invasive instrument and a steerable invasive instrument or two steerable instruments and one rigid instrument. It will be clear that the skilled person will realize that many combinations of steerable and rigid instruments are possible. However, for establishing triangulation which is one of the fundamental concepts of laparoscopic surgery, as it permits traction on tissue to facilitate dissection of it along normal anatomical planes, the assembly according to the invention requires the application of at least one steerable instrument together with the access device. The guiding arrangement is preferably a rigid tubular member comprising a number of lumen that each are adapted for receiving and guiding the invasive instruments of the assembly. The outer diameter of the tubular guiding arrangement is preferably adapted to the inner diameter of a standard trocar to establish a snug fit when they are combined for use in a surgical intervention.

The assembly according to the invention comprises a deflection setting arrangement for setting at least one angle between at least a part of the proximal end part and the rigid intermediate part of the steerable instrument. In this way, preferably an angle between a flexible zone that interconnects the rigid intermediate part and the proximal end part of the steerable instrument can be set. Because the steering arrangement of the steerable instrument according to the invention is adapted for translating a deflection of at least a part of the proximal end part relative to the rigid intermediate part into a related deflection of at least a part of the distal end part, the setting of the angle between the flexible zone that interconnects the rigid intermediate part and the proximal end part of the steerable instrument, results in the setting of a related angle preferably between a flexible zone that interconnects the rigid intermediate part and the distal end part of the steerable instrument.

In the context of this invention, the angle between the flexible zone that interconnects the rigid intermediate part and the distal end part of the steerable instrument is referred to as the triangulation angle. Despite the foregoing example, it will be appreciated that the skilled person understands that a related angle between any other flexible zone of the distal end part and the rigid intermediate part of the steerable instrument can be assumed as a result of setting an angle at the proximal end part of the steerable instrument, depending on the specific interconnections of flexible zones at the proximal and distal end parts of the steerable instrument. Furthermore, it is noted that the angle that is assumed at the distal end part of the instrument is related to the angle that is set at the proximal end part of the instrument in that the angle at the distal end part can be the same as the angle at the proximal end part and can also be larger or smaller than the angle at the proximal end part for example because of the use of amplifying or attenuating means.

Irrespective of the actual relationship between the angles at the proximal and distal end parts of the steerable instrument, i.e. equal, larger or smaller compared to each other, the deflection setting arrangement of the assembly according to the invention enables setting of the triangulation angle at the distal end of the steerable instrument by setting and fixing a deflection of at least a part of the proximal end part of the instrument relative to the rigid intermediate part.

Based on the above, it is clear that the assembly according to the invention obviates the need for arranging a conventional spreading means at the distal side of a conventional access device, which conventional spreading means in use of the conventional access device is located inside an object. Instead, the assembly according to the invention enables establishing triangulation by manipulation of the proximal end part of the steerable instrument of the assembly according to the invention. It will be clear for the skilled person that the access device of the assembly according to the invention has a less complicated construction and therefore brings about improved reliability and reduced cost. Despite its uncomplicated construction for establishing triangulation, the access device according to the invention provides the same capability as provided by conventional access devices with respect to controlling multiple degrees of freedom in which the steerable instrument and any associated tool arranged at its distal end part can move.

In the assembly according to the invention, the access device further comprises
a position setting arrangement that is adapted for setting a position of the invasive instrument relative to the access device in the guiding direction of the guiding arrangement; and optionally
a rotation setting arrangement that is adapted for setting a rotation of the invasive instrument about a rotation axis extending in the guiding direction of the guiding arrangement.

The position setting arrangement can be implemented using sliding means that engage with the invasive instruments. It will be appreciated that the skilled person will understand that the sliding means can be implemented using for example sliding arms that are connected with the guiding arrangement or any other suitable mechanism. An advantage of the assembly according to the invention is that the position setting arrangement allows setting of the position of the invasive instruments relative to the access device for each of the instruments individually. In this way, the position setting arrangement enables controllability of an important degree of freedom of the movement of the steerable instrument and the associated tool at its distal end.

An advantage of the assembly according to the invention is that the rotation setting arrangement allows setting of different working planes of the distal end parts of the steerable instruments and the associated tools for each of the instruments individually. In this way, the rotation setting arrangement enables controllability of another important degree of freedom of the movement of the steerable instrument and the associated tool at its distal end.

In another embodiment of the assembly according to the invention, the rotation setting arrangement comprises a pin and a frame having at least one receiving means for engaging with the pin. The receiving means can for example be a recess. If the frame comprises more than one recess, the recesses are preferably arranged according to a periphery of an imaginary cylinder that is coaxially arranged with a longitudinal axis of the rigid intermediate part of the instrument. The different recesses allow different rotation angles to be set upon engaging a specific recess with the pin.

The pin preferably extends in the guiding direction of the guiding arrangement. In this way, the rotation setting arrangement can also be used to guide the instrument, which is connected with the pin via the frame, in the guiding direction of the guiding arrangement.

In an embodiment of the assembly according to the invention, one of the pin and the frame is pivotably connected to the guiding arrangement via an arm. Preferably, the arm is arranged to allow one of the pin and the frame to be movable according to a periphery of an imaginary cylinder having a longitudinal axis that extends parallel to a longitudinal axis of the rigid intermediate part of the instrument. An advantage of this embodiment of the rotation setting arrangement is that the rotation angle can be changed without having to select a specific recess of the frame. Consequently, the frame would only need to have one recess. However, for redundancy reasons the frame could nevertheless be provided with more than one recess.

In an embodiment of the assembly according to the invention, the deflection setting arrangement comprises a bracket that is adapted for engaging with the steerable invasive instrument at either side of at least one flexible zone of the proximal end part of the steerable invasive instrument. Preferably, the bracket engages with the steerable invasive instrument at either side of a flexible zone that interconnects the rigid intermediate part and the proximal end part of the instrument. As explained above, in this way the triangulation angle can be set. It is noted, that despite the engagement of the bracket and the steerable instrument described above, the steerable instrument is rotatable around its own longitudinal axis.

Furthermore, it will be appreciated by the skilled person that depending on the specific interconnections of flexible zones at the proximal and distal end parts of the steerable instrument it is also possible that the bracket is adapted to engage with the steerable instrument at either side of a group of flexible zones at the proximal end thereby setting the triangulation angle.

In another embodiment of the assembly according to the invention, at least a part of the rotation setting arrangement and the deflection setting arrangement are integrated in a single component. In this way two degrees of freedom of the movement of the steerable instrument and the associated tool at its distal end can be controlled by a single part of the assembly according to the invention.

In the assembly according to the invention, the guiding arrangement comprises a rigid shaft having at least one lumen that is adapted for allowing the invasive instrument to pass through the shaft in the guiding direction of the shaft. Lumen that are not in use, i.e. that are not occupied by an instrument, can be closed by conventionally known closing means. By closing each lumen that is not in use, a leakage path for gasses that can be used to inflate a body cavity in which the surgical intervention is to take place, can be prevented.

In another embodiment of the assembly according to the invention, at least a proximal end part of the lumen has a tapered shape. An advantage of the tapered shape of the lumen is that an instrument with a tool, such as a forceps or a pair of scissors, arranged at its distal end part can be inserted easier into the lumen. For example, the jaw of a forceps can be in a slightly opened position that can complicate insertion of the forceps into the lumen, as the jaw first needs to be forced into a closed position as a result of an interaction between the forceps and the wall surrounding the access port of the lumen before the forceps and the instrument can be inserted into the lumen. Especially in situations in which speed of action is critical, an impeded insertion of an invasive instrument is a considerable disadvantage. Loss of valuable time can pose a threat to the human or animal under surgery. Furthermore, if fragile tools are being used, an impeded insertion could damage such tools. The tapered shape of the lumen can mitigate or at least reduce the abovementioned disadvantages. It will be appreciated by the skilled person that in addition to the proximal end part of the lumen, the distal end part of the lumen can have a tapered shape. The tapered shape of the lumen at the distal end part thereof could be beneficial for the insertion of a tool into the lumen upon retraction of the invasive instrument.

In another embodiment of the assembly according to the invention, a wall of the lumen is provided with at least one elongated recess extending along the length of the lumen. It can happen that tools even after having been brought into a position in which they have minimal dimensions, extend outside the inner diameter of the lumen. By providing some additional space in a radial direction of the lumen, the at least one elongated recess extending along the length of the lumen enables that these tools nevertheless can be inserted and passed through the lumen.

In another embodiment of the assembly according to the invention, an outer wall of the shaft is provided with at least one elongated recess extending along at least a part of the length of the shaft. In order to create sufficient space around the target area where the surgical intervention is to be taking place, a body cavity is usually being inflated using a gas such as carbon dioxide (CO₂). This gas is usually being fed into the body cavity via a clearance between the outer wall of a shaft of a conventional access device and the inner wall of a conventional trocar. A disadvantage of this approach is that a considerable clearance, in the order of several tenths of millimeters, is required. The at least one elongated recess that extends along at least a part of the length of the shaft at least reduces the latter disadvantage as it enables a considerably smaller clearance between an outer wall of a shaft of an access device according to the invention and the inner wall of a conventional trocar.

In another embodiment of the assembly according to the invention, the outer wall of the shaft is provided with at least one elongated slot that is arranged in an axial direction of the at least one lumen to provide an increased aperture of the at least one lumen at a distal end part of the lumen.

In another embodiment of the assembly according to the invention, the at least one elongated recess and the at least one elongated slot are arranged adjacent to each other in a circumferential direction of the shaft.

In another embodiment of the assembly according to the invention, the at least one elongated recess is arranged to extend at least equally far in an axial direction of the shaft towards a proximal end part of the shaft as the at least one elongated slot.

In another embodiment of the assembly according to the invention, the access device further comprises a first element of a coupling arrangement that is arranged at a proximal end of the shaft, and a capping element that comprises a second element of the coupling arrangement, the second element being connectable with the first element for connecting the capping element with the proximal end of the shaft, the capping element being provided with a first set of through holes comprising at least a first through hole that is arranged to provide a passage between a proximal end face and a distal end face of the capping element, the at least first through hole being arranged to enable insertion of the at least one invasive instrument into the at least one lumen.

In another embodiment of the assembly according to the invention, the at least first through hole at least partly has a tapered shape that is configured to narrow down towards the distal end face of the capping element.

In another embodiment of the assembly according to the invention, the first and second elements of the coupling arrangement are arranged to provide one of a bayonet coupling and a click-on coupling.

In another embodiment of the assembly according to the invention, the assembly further comprises at least a first sealing arrangement that is adapted for sealing the at least one lumen of the shaft in a fluid-tight manner.

In another embodiment of the assembly according to the invention, the at least first sealing arrangement is connected with the proximal end face of the shaft.

In another embodiment of the assembly according to the invention, the at least first sealing arrangement comprises a first member comprising a resilient material that is provided with a second set of through holes comprising at least a second through hole that is arranged to enable insertion of the at least one invasive instrument into the at least one lumen, the at least second through hole being arranged to define an inner surface of said resilient material surrounding the at least second through hole, the inner surface of the resilient material being adapted for enclosing at least a part of an outer surface of the proximal end part of the at least one invasive instrument to provide a fluid-tight seal for the at least one lumen.

In another embodiment of the assembly according to the invention, the sealing arrangement further comprises a second member that is connected with the first member, the second member comprising a rigid material that is provided with a first set of valves comprising at least a first valve that is arranged to enable insertion of the at least one invasive instrument into the at least one lumen when the at least first valve is in a first position and to provide a fluid-tight seal for the at least one lumen when the at least first valve is in a second position.

In another embodiment of the assembly according to the invention, the at least first valve comprises a resilient material that is provided with at least two intersecting slits that are arranged to define at least three valve parts that are adapted for allowing insertion of the at least one invasive instrument into the at least one lumen when the at least three valve parts are in a first position and providing a fluid-tight seal for the at least one lumen when the at least three valve parts are in a second position.

In another embodiment of the assembly according to the invention, the at least first sealing arrangement further comprises a third member that is arranged between the first member and the second member, the third member comprising a rigid material that is provided with a third set of through holes comprising at least a third through hole that is arranged to enable insertion of the at least one invasive instrument into the at least one lumen.

In another embodiment of the assembly according to the invention, the second member comprises at least a first element of an adjustable connecting arrangement and the third member comprises at least a second element of the adjustable connecting arrangement, the at least first element and the at least second element being configured to enable an adjustable connection of the second member and the third member to one another in a locked state, the first set of valves being arranged in alignment with the third set of through holes in the locked state to enable insertion of the at least one invasive instrument into the at least one lumen.

In another embodiment of the assembly according to the invention, the at least first element of the adjustable connecting arrangement comprises at least one recess and the at least second element of the adjustable connecting arrangement comprises at least one protrusion.

In another embodiment of the assembly according to the invention, the sealing arrangement is arranged between the capping element and the proximal end face of the shaft.

In another embodiment of the assembly according to the invention, the capping element and the sealing arrangement are arranged to form one integrated unit.

In another embodiment of the assembly according to the invention, the bracket comprises at least a first receiving space that is adapted for receiving at least a part of the proximal end part of the at least one invasive instrument, the bracket comprising a retaining member that is adapted for retaining the part of the invasive instrument within the receiving space.

In another embodiment of the assembly according to the invention, the bracket and the retaining member are arranged to form one integrated unit.

An aspect of the invention is an access device that is adapted for introducing at least one steerable invasive instrument comprising a proximal end part having at least one flexible zone into an object via an access port of the object, wherein the access device comprises
a rigid shaft having at least one lumen that is arranged to provide a passage between a proximal end face and a distal end face of the shaft; and
a deflection setting arrangement that comprises a bracket that is adapted for engaging with the steerable invasive instrument at either side of the flexible zone of the proximal end part of the steerable invasive instrument.

In an embodiment of the access device according to the invention, the access device further comprises
a position setting arrangement that is adapted for setting a position of the steerable invasive instrument relative to the shaft; and optionally
a rotation setting arrangement that is adapted for setting a rotation of the steerable invasive instrument about a rotation axis extending in the axial direction of the shaft.

In the access device according to the invention, the rotation setting arrangement comprises a pin and a frame having at least one receiving means, such as a recess, for engaging with the pin.

In the access device according to the invention, one of the pin and the frame is pivotably connected to the shaft via an arm.

In another embodiment of the access device according to the invention, at least a part of the rotation setting arrangement and the deflection setting arrangement are integrated in a single component.

In another embodiment of the access device according to the invention, at least a proximal end part of the lumen has a tapered shape.

In another embodiment of the access device according to the invention, a wall of the lumen is provided with at least one elongated recess extending along the length of the lumen.

In another embodiment of the access device according to the invention, an outer wall of the shaft is provided with at least one elongated recess extending along at least a part of the length of the shaft.

In another embodiment of the access device according to the invention, the outer wall of the shaft is provided with at least one elongated slot that is arranged in an axial direction of the at least one lumen to provide an increased aperture of the at least one lumen at a distal end part of the lumen.

In another embodiment of the access device according to the invention, the at least one elongated recess and the at least one elongated slot are arranged adjacent to each other in a circumferential direction of the shaft.

In another embodiment of the access device according to the invention, the at least one elongated recess is arranged to extend at least equally far in an axial direction of the shaft towards a proximal end part of the shaft as the at least one elongated slot.

In another embodiment of the access device according to the invention, the access device further comprises a first element of a coupling arrangement that is arranged at a proximal end of the shaft, and a capping element that comprises a second element of the coupling arrangement, the second element being connectable with the first element for connecting the capping element with the proximal end of the shaft, the capping element being provided with a first set of through holes comprising at least a first through hole that is arranged to provide a passage between a proximal end face and a distal end face of the capping element, the at least first through hole being arranged to enable insertion of the at least one invasive instrument into the at least one lumen.

In another embodiment of the access device according to the invention, the at least first through hole at least partly has a tapered shape that is configured to narrow down towards the distal end face of the capping element.

In another embodiment of the access device according to the invention, the first and second elements of the coupling arrangement are arranged to provide one of a bayonet coupling and a click-on coupling.

In another embodiment of the access device according to the invention, the access device further comprises at least a first sealing arrangement that is adapted for sealing the at least one lumen of the shaft in a fluid-tight manner.

In another embodiment of the access device according to the invention, the at least first sealing arrangement is connected with the proximal end face of the shaft.

In another embodiment of the access device according to the invention, the at least first sealing arrangement comprises a first member comprising a resilient material that is provided with a second set of through holes comprising at least a second through hole that is arranged to enable insertion of the at least one invasive instrument into the at least one lumen, the at least second through hole being arranged to define an inner surface of the resilient material surrounding the at least second through hole, the inner surface of the resilient material being adapted for enclosing at least a part of an outer surface of the proximal end part of the at least one invasive instrument to provide a fluid-tight seal for the at least one lumen.

In another embodiment of the access device according to the invention, the sealing arrangement further comprises a second member that is connected with the first member, the second member comprising a rigid material that is provided with a first set of valves comprising at least a first valve that is arranged to enable insertion of the at least one invasive instrument into the at least one lumen when the at least first valve is in a first position and to provide a fluid-tight seal for the at least one lumen when the at least first valve is in a second position.

In another embodiment of the access device according to the invention, the at least first valve comprises a resilient material that is provided with at least two intersecting slits that are arranged to define at least three valve parts that are adapted for allowing insertion of the at least one invasive instrument into the at least one lumen when the at least three valve parts are in a first position and providing a fluid-tight seal for the at least one lumen when the at least three valve parts are in a second position.

In another embodiment of the access device according to the invention, the at least first sealing arrangement further comprises a third member that is arranged between the first member and the second member, the third member comprising a rigid material that is provided with a third set of through holes comprising at least a third through hole that is arranged to enable insertion of the at least one invasive instrument into the at least one lumen.

In another embodiment of the access device according to the invention, the second member comprises at least a first element of an adjustable connecting arrangement and the third member comprises at least a second element of the adjustable connecting arrangement, the at least first element and the at least second element being configured to enable an adjustable connection of the second member and the third member to one another in a locked state, the first set of valves being arranged in alignment with the third set of through holes in the locked state to enable insertion of the at least one invasive instrument into the at least one lumen.

In another embodiment of the access device according to the invention, the at least first element of the adjustable connecting arrangement comprises at least one recess and the at least second element of the adjustable connecting arrangement comprises at least one protrusion.

In another embodiment of the access device according to the invention, the sealing arrangement is arranged between the capping element and the proximal end face of the shaft.

In another embodiment of the access device according to the invention, the capping element and the sealing arrangement are arranged to form one integrated unit.

In an of the access device according to the invention, the bracket comprises at least a first receiving space that is adapted for receiving at least a part of the proximal end part of the at least one invasive instrument, the bracket comprising a retaining member that is adapted for retaining the part of said invasive instrument within the receiving space.

In an of the access device according to the invention, the bracket and the retaining member are arranged to form one integrated unit.

In another embodiment, the access device comprises a sealing arrangement for use in an access device that is adapted for introducing at least one invasive instrument into an object via an access port of the object, the sealing arrangement comprising a first member that comprises a resilient material that is provided with a first set of valves comprising at least one valve, and at least a first element of an adjustable connecting arrangement, a second member comprising a rigid material that is provided with a first set of through holes comprising at least one through hole, and at least a second element of the adjustable connecting arrangement, the at least first element and the at least second element being configured to enable an adjustable connection of the first member and the second member to one another in a locked state, the first set of valves being arranged in alignment with the first set of through holes in the locked state.

In an embodiment of the sealing arrangement according to the invention, the at least first element of the adjustable connecting arrangement comprises at least one recess and the at least second element of the adjustable connecting arrangement comprises at least one protrusion.

In another embodiment of the sealing arrangement according to the invention, at least one valve of the first set of valves comprises a resilient material that is provided with at least two intersecting slits that are arranged to define at least three valve parts that are adapted for allowing passage of an invasive instrument when the at least three valve parts are in a first, at least partly opened position and providing a fluid-tight seal when the at least three valve parts are in a second, closed position.

In another embodiment of the sealing arrangement according to the invention,the sealing arrangement further comprises a third member that is connected with the second member, the third member comprising a resilient material that is provided with a second set of through holes that are arranged in alignment with the first set of through holes of the second member and the first set of valves of the first member in the locked state.

An aspect of the invention is an access device that is adapted for introducing at least one invasive instrument into an object via an access port of the object, wherein the access device comprises a rigid shaft having at least one lumen that is arranged to provide a passage between a proximal end face and a distal end face of the shaft, a capping element that is connectable with the proximal end face of the shaft, the capping element being provided with a first set of through holes comprising at least a first through hole that is arranged to provide a passage between a proximal end face and a distal end face of the capping element, the at least first through hole being arranged to enable insertion of the at least one invasive instrument into the at least one lumen, and a sealing arrangement according to the invention that is arranged between the capping element and the proximal end face of the shaft.

An aspect of the invention is an access device that is adapted for introducing at least one invasive instrument into an object via an access port of the object, wherein the access device comprises a rigid shaft having at least one lumen that is arranged to provide a passage between a proximal end face and a distal end face of the shaft, the shaft comprising an outer wall that is provided with at least one elongated recess that is arranged to extend along at least a part of the length of the shaft, the outer wall of the shaft further being provided with at least one elongated slot that is arranged in an axial direction of the at least one lumen to provide an increased aperture of the at least one lumen at a distal end part of the lumen.

In an embodiment of the access device according to the invention, the at least one elongated recess and the at least one elongated slot are arranged adjacent to each other in a circumferential direction of the shaft.

In an embodiment of the access device according to the invention, the at least one elongated recess is arranged to extend at least equally far in an axial direction of the shaft towards a proximal end part of the shaft as the at least one elongated slot.

An aspect of the invention is an assembly comprising at least one invasive instrument and an access device according to any combination of the embodiments described above.

The person skilled in the art will appreciate that advantageous effects of the various features of the above mentioned embodiments of the access device according to the invention are similar to the above described advantageous effects of similar features of the embodiments of the assembly according to the invention.

### BRIEF DESCRIPTION OF THE DRAWINGS

These and other features and effects of the present invention will be explained in more detail below with reference to drawings in which preferred and illustrative embodiments of the invention are shown. The person skilled in the art will realize that other alternatives and equivalent embodiments of the invention can be conceived and reduced to practice without departing from the scope of the present invention.
Figure 1a shows a schematic top view of a non-limiting embodiment of an assembly according to the invention. In this embodiment, the assembly comprises two steerable invasive instruments of which the distal end parts are triangulated as a result of the deflection of the proximal end parts of the instruments by the deflection setting arrangement of the access device.
Figure 1b shows a schematic perspective view of the embodiment of the assembly as shown in figure 1a .
Figure 1c shows a top view of the non-limiting embodiment of the assembly according to the invention as shown in figures 1a and 1b, wherein the tools that are arranged at the distal end parts are pointing towards each other.
Figures 1d shows a schematic perspective view of the embodiment of the assembly as shown in figure 1c.
Figure 2a shows a top view of a non-limiting embodiment of a rigid invasive instrument according to the invention.
Figure 2b shows a top view of a non-limiting embodiment of a steerable invasive instrument according to the invention.
Figure 2c provides a detailed perspective view of a non-limiting embodiment of the elongated tubular body of the steerable instrument.
Figure 2d provides a more detailed view of the distal end part of the elongated tubular body as shown in figure 2c.
Figure 2e shows a longitudinal cross-sectional view of the elongated tubular body of the steerable instrument as shown in figure 2c.
Figure 2f shows a longitudinal cross-sectional view of the elongated tubular body of the steerable instrument as shown in figure 2c, wherein the first proximal and first distal flexible zones are bent, thereby illustrating the operation of the steering arrangement.
Figure 2g shows a longitudinal cross-sectional view of the elongated tubular body of the steerable instrument as shown in figure 2f, wherein additionally the second proximal and second distal flexible zones are bent, thereby further illustrating the operation of the steering arrangement.
Figure 2h shows a perspective view of a part of the elongated tubular body as shown in figure 2c, wherein the outer cylindrical element partially has been removed to show an exemplary embodiment of the longitudinal steering elements that have been obtained after providing longitudinal slits to the wall of an intermediate cylindrical element that interconnects the first proximal flexible zone and the first distal flexible zone of the elongated tubular body.
Figure 2i shows a longitudinal cross-sectional view of an exemplary embodiment of a steerable instrument having one proximal and one distal flexible zone.
Figure 2j shows a perspective exploded view of the three cylindrical elements of the steerable instrument shown in figure 2i.
Figure 2k shows a top view of an unrolled version of an exemplary embodiment of the intermediate cylindrical element of the steerable instrument shown in figure 2j.
Figure 3a shows a schematic perspective view of a non-limiting embodiment of a bracket of the deflection setting arrangement of the assembly according to the invention.
Figure 3b shows a schematic side view of the bracket shown in figure 3a.
Figure 3c shows a schematic perspective view of another non-limiting embodiment of the bracket of the deflection setting arrangement.
Figure 3d shows a schematic side view of the bracket shown in figure 3c.
Figure 3e shows a schematic perspective view of yet another non-limiting embodiment of the bracket of the deflection setting arrangement.
Figure 3f shows a bottom view of the embodiment of the bracket shown in figure 3e.
Figure 4a shows a schematic perspective view of another non-limiting embodiment of the rotation setting arrangement of the access device according to the invention.
Figure 4b shows a schematic perspective view of the embodiment of the rotation setting arrangement of the access device shown in figure 4a, wherein the arms of the rotation setting arrangement are in different positions with respect to each other.
Figure 5a shows a top view of another non-limiting embodiment of the position setting arrangement of the assembly according to the invention.
Figure 5b shows a cross-sectional view of the embodiment of the access device shown in figure 5a.
Figure 6a shows a combination of a schematic perspective view and a longitudinal cross-sectional view of a non-limiting embodiment of a part of the rigid shaft of the access device according to the invention.
Figure 6b shows a combination of a schematic perspective view and a longitudinal cross-sectional view of another non-limiting embodiment of a part of the rigid shaft of the access device according to the invention.
Figure 6c shows a schematic perspective view of yet another non-limiting embodiment of a part of the rigid shaft of the access device according to the invention.
Figure 6d shows a schematic side view of another non-limiting embodiment of the rigid shaft of the access device according to the invention.
Figure 7a shows a schematic cross-sectional view of a non-limiting embodiment of the proximal end of the shaft that is connected with a capping element and a sealing arrangement according to the invention.
Figure 7b shows a schematic exploded view of a non-limiting embodiment of a sealing arrangement according to the invention.
Figure 7c shows a schematic perspective view of a non-limiting embodiment of a sealing arrangement according to the invention and especially of a non-limiting embodiment of the second member of the sealing arrangement.
Figure 8a shows a schematic top view of non-limiting embodiments of both the capping element and a bracket according to the invention.
Figure 8b shows a schematic perspective view of another non-limiting embodiment of the access device according to the invention.

The figures are not necessarily drawn to scale. In the figures identical components are denoted by the same reference numerals.

### DETAILED DESCRIPTION OF EMBODIMENTS

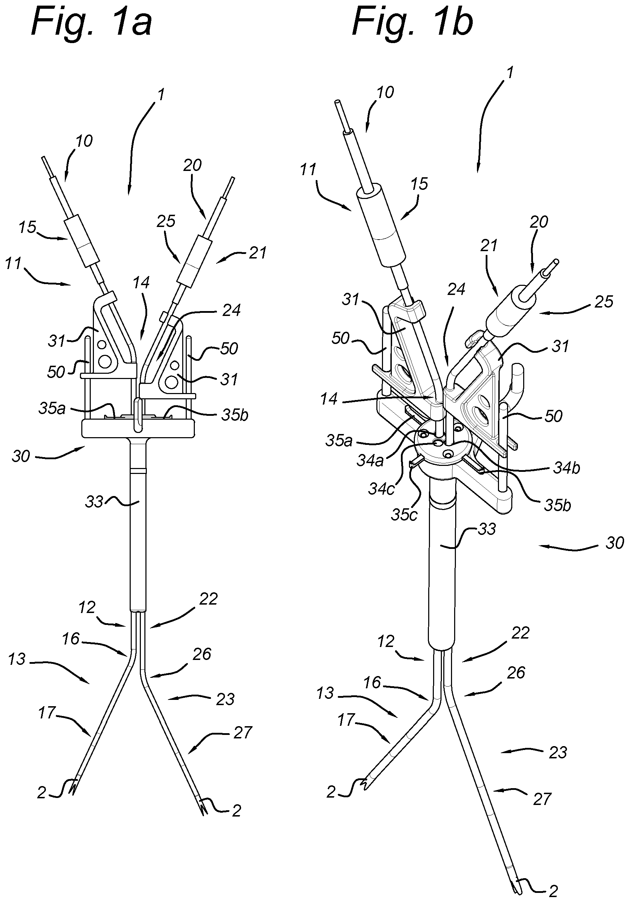

Figure 1a shows a schematic top view of a non-limiting embodiment of an assembly 1 according to the invention. In this embodiment the assembly 1 comprises two non-limiting identical steerable invasive instruments 10, 20 and a non-limiting embodiment of an access device 30. It will be appreciated by the skilled person that in another embodiment of the assembly 1 according to the invention, non-identical invasive instruments can be used. For example, a rigid instrument and a steerable instrument or two steerable instruments both equipped with different tools can be used. In the following, firstly details of non-limiting embodiments of invasive instruments of the assembly according to the invention will be discussed. Secondly, details of non-limiting embodiments of the access device of the assembly according to the invention will be discussed.

### Invasive instruments

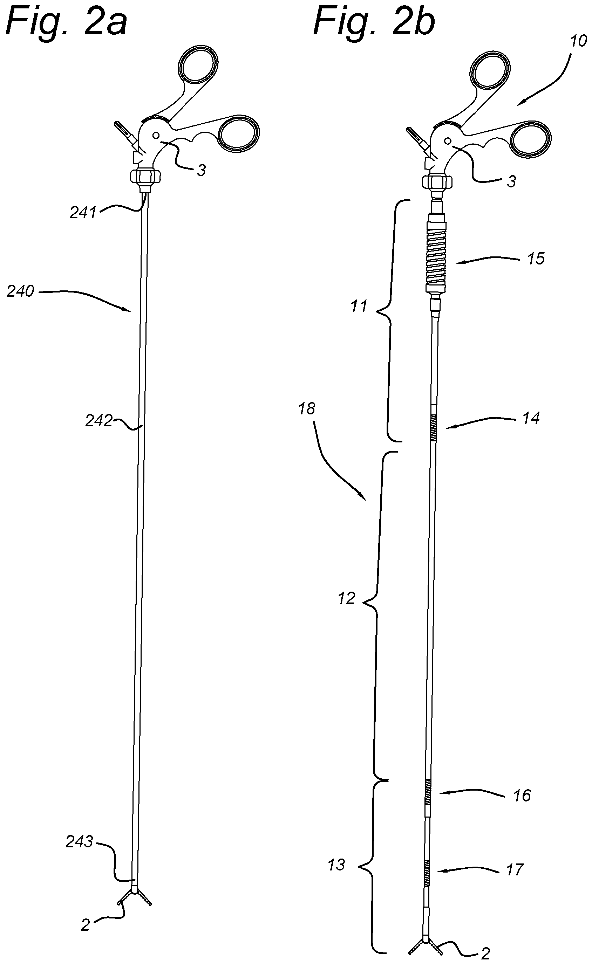

Figure 2a shows a top view of a non-limiting embodiment of a rigid invasive instrument 240. Details of the non-limiting embodiment of the steerable invasive instruments 10, 20 as shown in figure 1a are explained for one of them in relation to figures 2b-2k.

The rigid invasive instrument 240 as shown in figure 2a comprises an elongated shaft 242 having a proximal end part 241 and a distal end part 243. At the distal end part 243 a tool 2, for example a forceps, is arranged. At the proximal end part 241 a handle 3 is arranged that is adapted for manipulating the tool 2, i.e. opening and closing the jaw of the forceps.

Figure 2b shows a top view of one of the identical steerable invasive instruments 10, 20 as shown in figure 1a. The steerable instrument 10 comprises an elongated tubular body 18 having a proximal end part 11 including two flexible zones 14, 15, a distal end part 13 including two flexible zones 16, 17, and a rigid intermediate part 12. At the distal end part 13 a forceps 2 is arranged. At the proximal end part 11 a handle 3 is arranged that is adapted for opening and closing the jaw of the forceps 2.

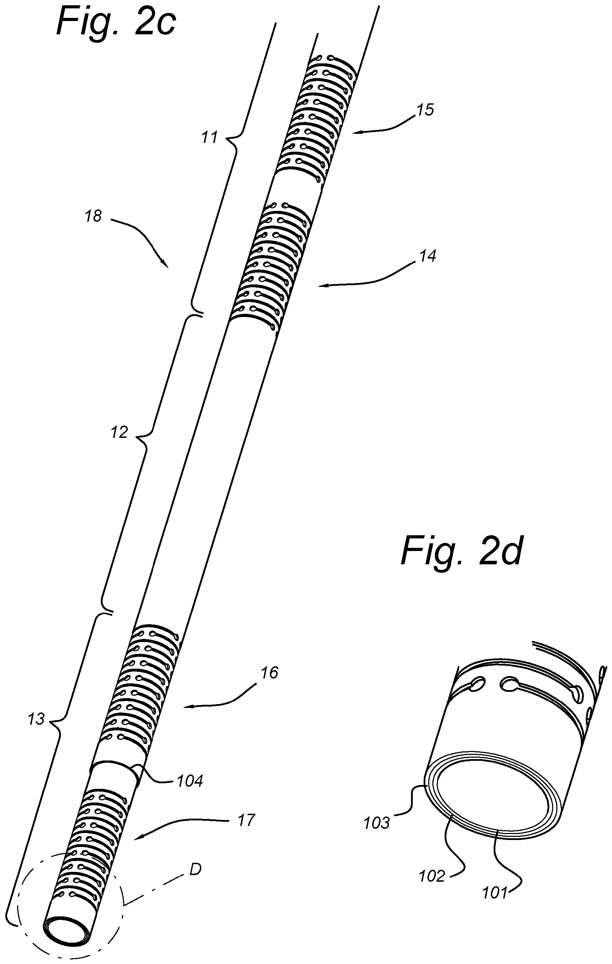

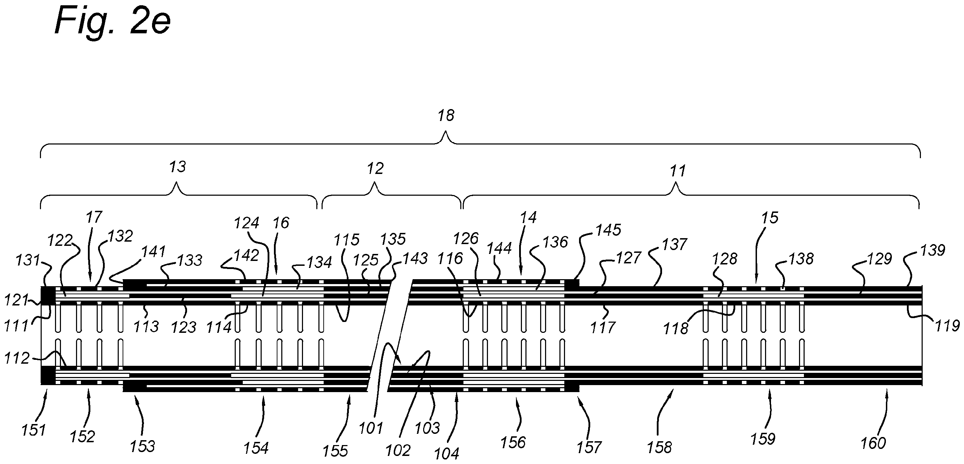

Figure 2c provides a detailed perspective view of the elongated tubular body 18 of the steerable instrument 10 and shows that the elongated tubular body 18 comprises of a number of co-axially arranged layers or cylindrical elements including an outer cylindrical element 104 that ends after the first flexible zone 16 at the distal end 13. Figure 2d provides a more detailed view of the distal end part 13 and shows that it includes three co-axially arranged layers or cylindrical elements being an inner cylindrical element 101, a first intermediate cylindrical element 102 and a second intermediate cylindrical element 103. It will be clear to the skilled person that the elongated tubular body 18 as shown in figure 2c comprises four cylindrical elements in total. The elongated tubular body 18 according to the embodiment shown in figure 2c comprises two intermediate cylindrical elements 102 and 103 in which the steering members of the steering arrangement are arranged. The steering arrangement in the exemplary embodiment of the elongated tubular body 18 as shown in figure 2c comprises the two flexible zones 14, 15 at the proximal end part 11 of the elongated tubular body 18, the two flexible zones 16, 17 at the distal end part 13 of the elongated tubular body 18 and the steering members that are arranged between related flexible zones at the proximal 11 and distal 13 end parts. The actual arrangement of the steering members is shown in figure 2e which provides a schematic longitudinal cross-sectional view of the exemplary embodiment of the elongated tubular body 18 as shown in figure 2c.

Figure 2e shows the four layers or cylindrical elements mentioned above, i.e. the inner cylindrical element 101, the first intermediate cylindrical element 102, the second intermediate cylindrical element 103, and the outer cylindrical element 104.

The inner cylindrical element 101, as seen along its length, comprises a rigid ring 111, which is arranged at the distal end part 13 of the steerable instrument 10, a first flexible portion 112, a first intermediate rigid portion 113, a second flexible portion 114, a second intermediate rigid portion 115, a third flexible portion 116, a third intermediate rigid portion 117, a fourth flexible portion 118, and a rigid end portion 119, which is arranged at the proximal end portion 11 of the steerable instrument 10.

The first intermediate cylindrical element 102, as seen along its length, comprises a rigid ring 121, a first flexible portion 122, a first intermediate rigid portion 123, a second flexible portion 124, a second intermediate rigid portion 125, a third flexible portion 126, a third intermediate rigid portion 127, a fourth flexible portion 128, and a rigid end portion 129. The longitudinal dimension of the rigid ring 121, the first flexible portion 122, the first intermediate rigid portion 123, the second flexible portion 124, the second intermediate rigid portion 125, the third flexible portion 126, the third intermediate rigid portion 127, the fourth flexible portion 128, and the rigid end portion 129 are approximately equal to the longitudinal dimension of the rigid ring 111, the first flexible portion 112, the first intermediate rigid portion 113, the second flexible portion 114, the second intermediate rigid portion 115, the third flexible portion 116, the third intermediate rigid portion 117, the fourth flexible portion 118, and the rigid end portion 119, respectively and are coinciding with these portions as well.

The second intermediate cylindrical element 103, as seen along its length, comprises a first rigid ring 131, a first flexible portion 132, a second rigid ring 133, a second flexible portion 134, a first intermediate rigid portion 135, a first intermediate flexible portion 136, a second intermediate rigid portion 137, a second intermediate flexible portion 138, and a rigid end portion 139. The longitudinal length of the first rigid ring 131, the first flexible portion 132 together with the second rigid ring 133 and the second flexible portion 134, the first intermediate rigid portion 135, the first intermediate flexible portion 136, the second intermediate rigid portion 137, the second intermediate flexible portion 138, and the rigid end portion 139 are approximately equal to the longitudinal dimension of the rigid ring 111, the first flexible portion 112, the first intermediate rigid portion 113, the second flexible portion 114, the second intermediate rigid portion 115, the third flexible portion 116, the third intermediate rigid portion 117, the fourth flexible portion 118, and the rigid end portion 119, respectively and are coinciding with these portions as well.

The outer cylindrical element 104, as seen along its length, comprises a first rigid ring 141, a first flexible portion 142, a first intermediate rigid portion 143, a second flexible portion 144, and a second rigid ring 145. The longitudinal length of the first flexible portion 142, the first intermediate rigid portion 143 and the second flexible portion 144 are approximately equal to the longitudinal dimension of the second flexible portion 134, the first intermediate rigid portion 135 and the first intermediate flexible portion 136, respectively and are coinciding with these portions as well. The rigid ring 141 has approximately the same length as the rigid ring 133 and is connected thereto. Preferably, the rigid ring 145 overlaps with the second intermediate rigid portion 137 only over a length that is required to make an adequate connection between the rigid ring 145 and the second intermediate rigid portion 137, respectively. The end faces of the rigid rings 111, 121 and 131 can be connected to each other and the same applies to the end faces of the rigid end portions 119, 129 and 139.

The inner and outer diameters of the cylindrical elements 101, 102, 103, and 104 are chosen in such a way that the outer diameter of inner cylindrical element 101 is almost equal to the inner diameter of the first intermediate cylindrical element 102, the outer diameter of the first intermediate cylindrical element 102 is almost equal to the inner diameter of the second intermediate cylindrical element 103 and the outer diameter of the second intermediate cylindrical element 103 is almost equal to the inner diameter of the outer cylindrical element 104, in such a way that a sliding movement of the adjacent cylindrical elements with respect to each other is possible.

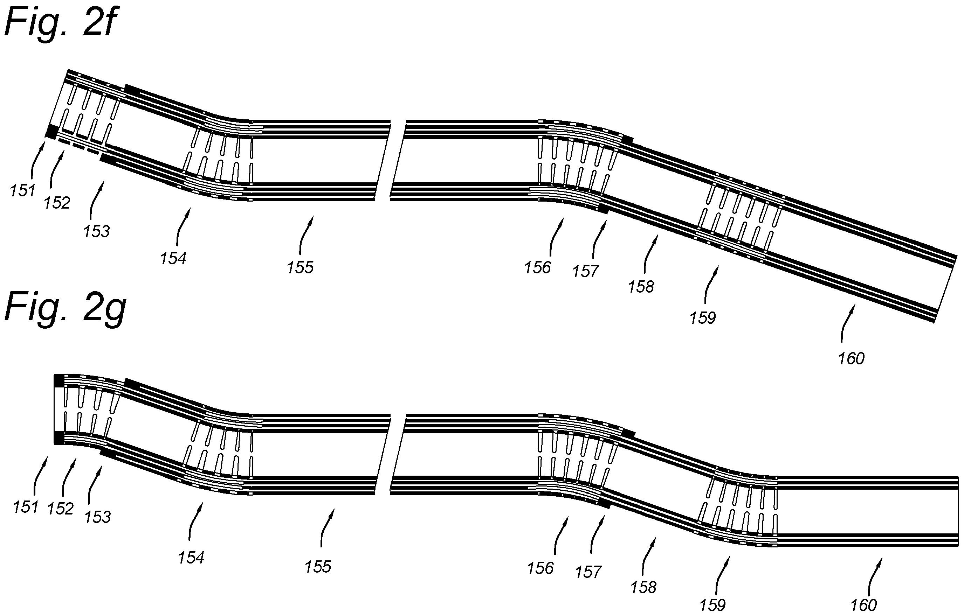

As can be seen in figure 2e, flexible zone 14 of the proximal end part 11 is connected to the flexible zone 16 of the distal end part 13 by portions 134, 135 and 136, of the second intermediate cylindrical element 103, which form a first set of longitudinal steering members of the steering arrangement of the steerable instrument 10. Furthermore, flexible zone 15 of the proximal end part 11 is connected to the flexible zone 17 of the distal end part 13 by portions 122, 123, 124, 125, 126, 127, and 128 of the first intermediate cylindrical element 102, which form a second set of longitudinal steering members of the steering arrangement. The use of the construction as described above allows the steerable instrument 10 to be used for double bending. The working principle of this construction will be explained with respect to the examples shown in figures 2f and 2g.

For the sake of convenience, the different portions of the cylindrical elements 101, 102, 103, and 104 have been grouped into zones 151 - 160 that are defined as follows. Zone 151 comprises the rigid rings 111, 121, and 131. Zone 152 comprises the portions 112, 122, and 132. Zone 153 comprises the rigid rings 133 and 141 and the portions 113 and 123. Zone 154 comprises the portions 114, 124, 134 and 142. Zone 155 comprises the portions 115, 125, 135 and 143. Zone 156 comprises the portions 116, 126, 136 and 144. Zone 157 comprises the rigid ring 145 and the parts of the portions 117, 127, and 137 coinciding therewith. Zone 158 comprises the parts of the portions 117, 127, and 137 outside zone 157. Zone 159 comprises the portions 118, 128 and 138. Finally, zone 160 comprises the rigid end portions 119, 129 and 139.

In order to deflect at least a part of the distal end part 13 of the steerable instrument 10, it is possible to apply a bending force, in any radial direction, to zone 158. According to the examples shown in figures 2f and 2g, zone 158 is bent downwards with respect to zone 155. Consequently, zone 156 is bent downwards. Because of the first set of steering members comprising portions 134, 135, and 136 of the second intermediate cylindrical element 103 that are arranged between the second intermediate rigid portion 137 and the second rigid ring 133, the downward bending of zone 156 is transferred by a longitudinal displacement of the first set of steering members into an upward bending of zone 154 with respect to zone 155. This is shown in both figures 2f and 2g.

It is to be noted that the exemplary downward bending of zone 156, only results in the upward bending of zone 154 at the distal end of the instrument as shown in figure 2f. Bending of zone 152 as a result of the bending of zone 156 is prevented by zone 153 that is arranged between zones 152 and 154. When subsequently a bending force, in any radial direction, is applied to the zone 160, zone 159 is also bent. As shown in figure 2g, zone 160 is bent in an upward direction with respect to its position shown in figure 2f. Consequently, zone 159 is bent in an upward direction. Because of the second set of steering members comprising portions 122, 123, 124, 125, 126, 127 and 128 of the first intermediate cylindrical element 102 that are arranged between the rigid ring 121 and the rigid end portion 129, the upward bending of zone 159 is transferred by a longitudinal displacement of the second set of steering members into a downward bending of zone 152 with respect to its position shown in figure 2f.

Figure 2g further shows that the initial bending of the instrument in zone 154 as shown in figure 2f will be maintained because this bending is only governed by the bending of zone 156, whereas the bending of zone 152 is only governed by the bending of zone 159 as described above. Due to the fact that zones 152 and 154 are bendable independently with respect to each other, it is possible to give the distal end part 13 of the steerable instrument 10 a position and longitudinal axis direction that are independent from each other. In particular the distal end part 13 can assume an advantageous S-like shape. In known instruments such as described in EP-A-1 708 609, the position and the direction of the longitudinal axis are always coupled and cannot be individually controlled. The skilled person will appreciate that the capability to independently bend zones 152 and 154 with respect to each other, significantly enhances the maneuverability of the distal end part 13 and therefore of the steerable instrument 10 as a whole.

Obviously, it is possible to vary the lengths of the flexible portions shown in figures 2e-2g as to accommodate specific requirements with regard to bending radii and total lengths of the distal end part 13 and the proximal end part 11 of the steerable instrument 10 or to accommodate amplification or attenuation ratios between bending of at least a part of the proximal end part 11 and at least a part of the distal end part 13.

The steering arrangement of the steerable invasive instrument 10 may comprise conventional steering cables as steering members. However due to well-known disadvantages of conventional steering cables, the steering members preferably comprise one or more sets of longitudinal elements that form integral parts of the one or more intermediate cylindrical elements 102, 103. Preferably, the longitudinal elements comprise remaining parts of the wall of an intermediate cylindrical element 102, 103 after the wall of the intermediate cylindrical element 102, 103 has been provided with longitudinal slits that define the remaining longitudinal steering elements.

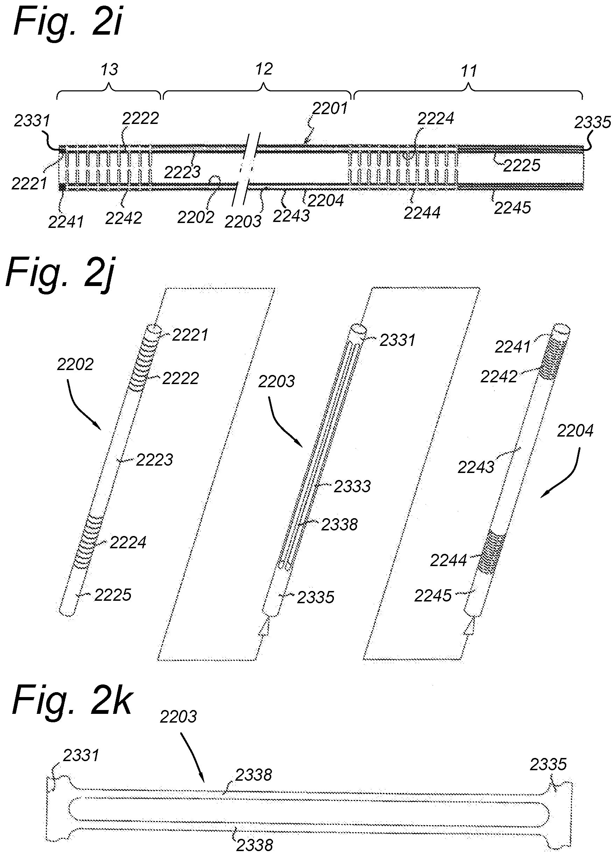

Further details regarding the fabrication of the latter longitudinal steering elements are provided with reference to figures 2i-2k regarding an exemplary embodiment of a steerable instrument that comprises only one flexible zone at both its proximal 11 and distal end 13 parts.

Figure 2i shows a longitudinal cross-section of a steerable instrument 2201 comprising three co-axially arranged cylindrical elements, i.e. inner cylindrical element 2202, intermediate cylindrical element 2203 and outer cylindrical element 2204. The inner cylindrical element 2202 comprises a first rigid end part 2221, which is located at the distal end part 13 of the instrument 2201, a first flexible part 2222, an intermediate rigid part 2223, a second flexible part 2224 and a second rigid end part 2225, which is located at the proximal end part 11 of the instrument 2201.

The outer cylindrical element 2204 also comprises a first rigid part 2241, a first flexible part 2242, an intermediate rigid part 2243, a second flexible part 2244 and a second rigid part 2245. The lengths of the different parts of the cylindrical elements 2202 and 2204 are substantially the same so that when the cylindrical element 2202 is inserted into the cylindrical element 2204, the different parts are positioned against each other. The intermediate cylindrical element 2203 also has a first rigid part 2231 and a second rigid end part 2235 which in the assembled condition are located between the corresponding rigid parts 2221, 2241 and 2225, 2245 respectively of the two other cylindrical elements. The intermediate part 2333 of the intermediate cylindrical element 2203 comprises three or more separate longitudinal elements which can have different forms and shapes as will be explained below. After assembly of the three cylindrical elements 2202, 2203 and 2204 whereby the element 2202 is inserted in the element 2203 and the two combined elements 2202, 2203 are inserted into the element 2204, the end faces of the three cylindrical elements 2202, 2203 and 2204 are connected to each other at both ends so as to have one integral unit.

In the embodiment shown in figure 2j the intermediate part 2333 of intermediate cylindrical element 2203 comprises a number of longitudinal elements 2338 with a uniform cross-section so that the intermediate part 2333 has the general shape and form as shown in the unrolled condition of the intermediate cylindrical element 2203 in figure 2k. From figure 2k it also becomes clear that the intermediate part 2333 is formed by a number of over the circumference of the intermediate cylindrical part 2203 equally spaced parallel longitudinal elements 2338. Advantageously, the number of longitudinal elements 2338 is at least three, so that the instrument 2201 becomes fully controllable in any direction, but any higher number is possible as well. The production of such an intermediate part is most conveniently done by injection moulding or plating techniques or starting from a cylindrical tube with the desired inner and outer diameters and removing parts of the wall of the cylindrical tube required to end up with the desired shape of the intermediate cylindrical element 2203.

The removal of material can be done by means of different techniques such as laser cutting, photochemical etching, deep pressing, conventional chipping techniques such as drilling or milling, high pressure water jet cutting systems or any suitable material removing process available. Preferably, laser cutting is used as this allows for a very accurate and clean removal of material under reasonable economic conditions. The above mentioned processes are convenient ways as the member 2203 can be made so to say in one process, without requiring additional steps for connecting the different parts of the intermediate cylindrical member as required in the conventional instruments, where conventional steering cables must be connected in some way to the end parts. The same type of technology can be used for producing the inner and outer cylindrical elements 2202 and 2204 with their respective flexible parts 2222, 2224, 2242 and 2244.

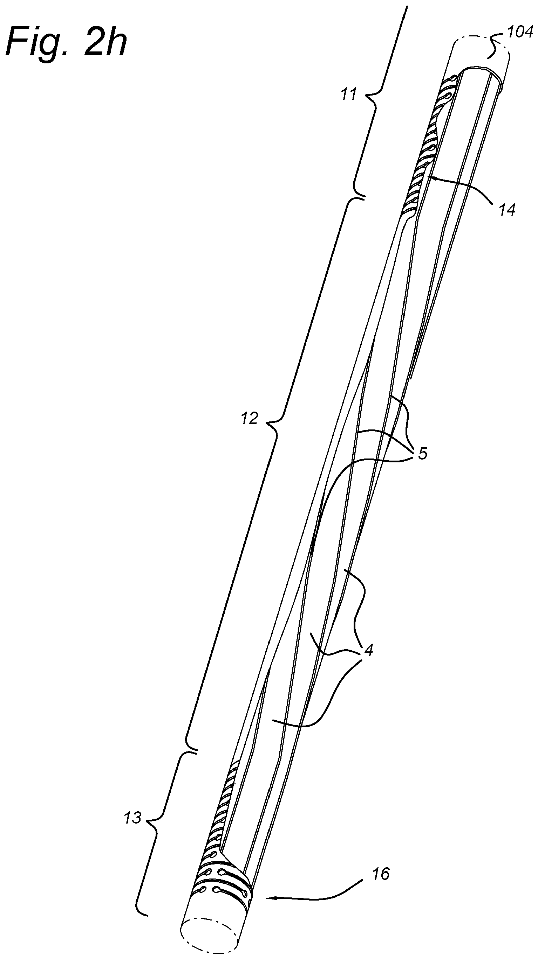

Figure 2h shows an exemplary embodiment of longitudinal steering elements 4 that have been obtained after providing longitudinal slits 5 to the wall of the second intermediate cylindrical element 103 that interconnects proximal flexible zone 14 and distal flexible zone 16 as described above.

The flexible portions 112, 132, 114, 142, 116, 144, 118, and 138 as shown in figure 2e can be obtained by the methods described in the European patent application 08 004 373.0 filed on 10.03.2008, page 5, lines 15-26, but any other suitable process can be used to make flexible portions.

Furthermore, if the portions 122, 123, 124, 125, 126, 127, and 128 of the first intermediate cylindrical element 102 and the portions 134, 135, and 136 of the second intermediate cylindrical element 103 that respectively form the first and second set of longitudinal steering members, as shown in figure 2e, are implemented as longitudinal steering elements 4 as shown in figure 2h, the fabrication methods described above can be used.

Otherwise, the longitudinal elements 4 can also be obtained by any other technique known in the art such as for example described in EP-A-1 708 609. The only restriction with respect to the construction of the longitudinal elements 4 used in these portions is that the total flexibility of the instrument in these locations where the flexible portions coincide must be maintained.

The different co-axially arranged layers or cylindrical elements 101, 102, 103, 104, 2202, 2203, and 2204 as described above in relation to the exemplary embodiments of the steerable instruments shown in figures 2e and 2i respectively, may be produced by any of the known methods, provided that they are suitable to make a multilayer system. A multilayer system is to be understood as being a steerable instrument that comprises at least two separate sets of longitudinal elements for transferring the movement of the proximal end part to the distal end part. The assembly of the different cylindrical elements can be realized in the same way as well.

### Access device

Figure 1a further shows a non-limiting embodiment of an access device 30 of the assembly 1 according to the invention. The access device 30 comprises a guiding arrangement 33 that is adapted for receiving and guiding the steerable instruments 10, 20 into an object in a guiding direction thereof. The object can be a human or animal body but can also be a piece of mechanical and/or electronic hardware.

Figure 1b shows a perspective view of the same non-limiting embodiment of the assembly 1 as shown in figure 1a. In this way a better view is provided on the lumen 34a and 34b in which the steerable instruments 10, 20 are arranged. In the non-limiting embodiment of the access device 30 shown in figure 1b, the guiding arrangement is a rigid shaft 33 that comprises three visible lumen 34a, 34b, and 34c. The number of lumen can be any required number depending on the type of intervention and the number of instruments that are required to perform this intervention. As shown in figure 1a and 1b, the lumen 34a and 34b are occupied by the steerable instruments 10, 20 respectively, wherein the instruments 10, 20 are guided in a guiding direction of the shaft 33. The unoccupied lumen 34c is closed off by a known closing means 35c. Examples of known closing means are shutters or valves. The closing means can prevent a leakage path for gasses out of the object in which an intervention is to take place. The closing means shown in figure 1b is a manually operable fluid-tight shutter 35c.

As the two steerable instruments 10, 20 are guided via the shaft 33, the intermediate rigid parts 12, 22 of the steerable instruments 10, 20 are located closely to each other in a radial direction of the shaft 33. Typical heart-to-heart distances between the lumen 34a and 34b are in a range of 2mm - 15mm. The skilled person will appreciate that the actual value depends among others on the type of intervention. As a result, triangulation of the distal end parts 13, 23 is required in order to provide sufficient distance between the distal end parts 13, 23 to enable comfortable maneuvering and positioning of the tools 2 that are arranged at the distal end parts 13, 23 at the operation site. Furthermore, triangulation of the distal end parts 13, 23 can reduce the risk of clashing of the distal end parts 13, 23 and the associated tools 2. At the proximal side of the access device 30, the proximal end parts 11, 21 also need to be spaced apart in order to allow for comfortable maneuvering of the control handles 3 of the steerable instruments 10, 20.

From figures 2f and 2g and the corresponding description provided above, the skilled person will appreciate that a deflection of at least a part of the proximal end part 11 in any radial direction of the instrument can be translated to a deflection of at least a part of the distal end part 13 in an opposite radial direction. However, a deflection in the same radial direction of at least a part of the proximal and distal end parts is also possible. This can be achieved by arranging the longitudinal steering members of the steering arrangement under an angle of twist that is equal to 180° as will be explained in more detail below.

In order to achieve triangulation of the distal end parts 13, 23 of the steerable invasive instruments 10, 20 as shown in figures 1a-1d, at least the longitudinal steering members that interconnect the flexible zones 14 and 16 have to be arranged under an angle of twist. Such an arrangement of the longitudinal steering members is shown in figure 2h from which it can be observed that the longitudinal elements 4 that interconnect the proximal flexible zone 14 and the distal flexible zone 16 are arranged under an angle of twist.

In the context of this invention, the angle of twist is defined as the angle along the circumference of the elongated tubular body between a first location of a first end part of a longitudinal element on the elongated tubular body and a second location of a second end part of the longitudinal element on the elongated tubular body. In the exemplary embodiment of a part of the elongated tubular body 18 shown in figure 2h, the angle of twist is defined as the angle along the circumference of the intermediate cylindrical element 103 of the elongated tubular body 18 of the steerable instrument 10 between the location of a first end part of a longitudinal element 4 at the proximal flexible zone 14 of the elongated tubular body 18 and the location of a second end part of the longitudinal element 4 at the distal flexible zone 16 of the elongated tubular body 18. The angle of twist can be any angle out of 0°- 360°.

If the angle of twist is equal to 180°, a radial deflection of the distal flexible zone 16 as a result of a radial deflection of the proximal flexible zone 14, is in the same direction with respect to the rigid intermediate part 12 of the elongated tubular body 18. This relationship between the radial deflection of the proximal and distal end parts is required for establishing triangulation of the distal end parts 13, 23 of the steerable instruments 10, 20 of the assembly 1 according to the invention as shown in figures 1a-1d. If the angle of twist is equal to 0°, the radial deflection of a distal flexible zone as a result of a radial deflection of a proximal flexible zone is in an opposite direction as shown in figures 2f and 2g.

Figures 1a-1d show that triangulation of the distal end parts 13, 23 is established by deflecting the proximal end parts 11, 21 of the instruments 10, 20 using deflection setting arrangements 31 that are arranged at the proximal side of the access device 30. Contrary to known access devices, the access device 30 of the assembly 1 according to the invention obviates the use of conventional spreading means that are arranged at the distal side of the access device, wherein the spreading means are operated by a control means that is arranged at the proximal side of the access device. Well-known examples of conventional spreading means are a controllable wedge and an inflatable-deflatable balloon. Other known means to achieve triangulation of the distal end parts of invasive instruments in general are the use of spreader arms arranged near the distal end parts of the steerable instruments and the use of pre-bent or steerable guiding tubes and/or endoscopes.

The assembly 1 according to the invention comprises a deflection setting arrangement 31 that is adapted for setting at least one angle between at least a part of the proximal end part 11 and the rigid intermediate part 12 of a steerable instrument 10. In this way, preferably an angle between the flexible zone 14 that interconnects the rigid intermediate part 12 and the proximal end part 11 of the steerable instrument 10 can be set. Because the steering arrangement of the steerable instrument 10 according to the invention is adapted for translating a deflection of at least a part of the proximal end part 11 relative to the rigid intermediate part 12 into a related deflection of at least a part of the distal end part 13, the setting of the angle between the flexible zone 14 that interconnects the rigid intermediate part 12 and the proximal end part 11 of the steerable instrument 10, results in the setting of a related angle preferably between the flexible zone 16 that interconnects the rigid intermediate part 12 and the distal end part 13 of the steerable instrument 10.

In the context of this invention, the angle between the flexible zone 16 that interconnects the rigid intermediate part 12 and the distal end part 13 of the steerable instrument 10 is referred to as the triangulation angle. Despite the foregoing example, it will be appreciated that the skilled person understands that a related angle between any other flexible zone of the distal end part 13 and the rigid intermediate part 12 of the steerable instrument 10 can be assumed as a result of setting an angle at the proximal end part 11 of the steerable instrument 10. This depends on the specific interconnections of flexible zones at the proximal and distal end parts of the steerable instrument. Furthermore, it is noted that the angle that is assumed at the distal end part 13 of the instrument is related to the angle that is set at the proximal end part 11 of the instrument because the angle at the distal end part 13 can be the same as the angle at the proximal end part and it can be larger or smaller than the angle at the proximal end part 11 for example because of the use of amplifying or attenuating means.

Irrespective of the actual relationship between the angles at the proximal and distal end parts of the steerable instrument, i.e. equal, larger or smaller compared to each other, the deflection setting arrangement 31 of the assembly 1 according to the invention enables setting of the triangulation angle at the distal end part 13 of the steerable instrument by setting and fixing a deflection of at least a part of the proximal end part 11 of the instrument 10 relative to the rigid intermediate part 12.

Based on the above, it is clear that the assembly 1 according to the invention obviates the need for arranging a conventional spreading means as mentioned above at the distal side of the access device, which conventional spreading means in use of a conventional access device is located inside the object under surgery. Instead, the assembly 1 according to the invention enables establishing triangulation by manipulation of the proximal end part 11 of the steerable instrument 10 of the assembly 1 according to the invention. It will be clear for the skilled person that the access device 30 of the assembly 1 according to the invention has a less complicated construction and therefore brings about improved reliability and reduced cost.

Despite its uncomplicated construction for establishing triangulation, the access device 30 according to the invention provides the same capability as provided by conventional access devices with respect to controlling multiple degrees of freedom in which the steerable instrument 10 and any associated tool 2 arranged at its distal end part 13 can move. Furthermore, a less complicated construction of the access device 30 can be beneficial with respect to sterilization issues.

Figures 1a-1d show a non-limiting embodiment of the deflection setting arrangement 31 comprising a bracket that is in engaging contact with the steerable instrument 10, 20 at either side of the proximal flexible zone 14, 24 that interconnects the rigid intermediate part 12, 22 and the proximal end part 11, 21 of the instrument 10, 20. As explained above, in this way the triangulation angle can be set. In this way, one degree of freedom regarding the movement of the steerable instruments 10, 20 can be controlled by the assembly 1 according to the invention. After having set the appropriate triangulation angle, the operator does not need to pay attention to this aspect anymore. Hence, the operator can focus on executing the intervention.

It is noted, that despite the engagement of the brackets 31 and the steerable instrument 10, 20 described above, the steerable instrument 10, 20 are individually rotatable around their own longitudinal axes. Furthermore, it will be appreciated by the skilled person that depending on the specific interconnections of flexible zones arranged at the proximal and distal end parts of the steerable instrument respectively, it is also possible that the bracket 31 is adapted to engage with the steerable instrument 10, 20 at either side of a group of flexible zones at the proximal end part 11 thereby setting the triangulation angle.

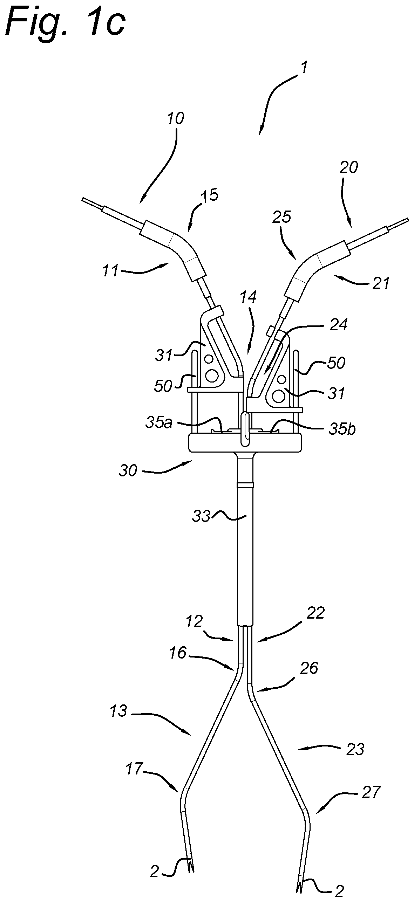

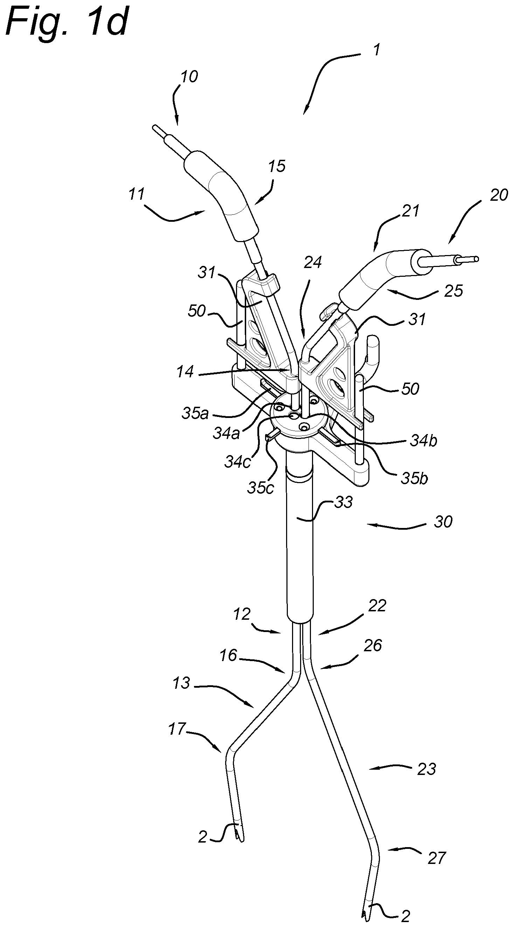

Figures 1c and 1d show that the tools 2 that are arranged at the distal end parts 13, 23 can be positioned such that they point towards each other as a result of bending the second proximal flexible zones 15, 25 in opposite directions with respect to each other. Based on the foregoing, it will be clear to the skilled person that the sets of longitudinal steering members that are interconnecting the second proximal flexible zones 15, 25 and the second distal flexible zones 17, 27 in both instruments 10, 20 are not arranged under an angle of twist, or said differently the angle of twist of these sets of steering members is equal to 0°. A first advantage of the latter relationship between the radial deflections of the proximal and distal flexible zones is that clashing of handles that are arranged at the proximal end part of the elongated tubular body can be avoided. A second advantage is that the operator has a user experience regarding the manipulation of the steerable instruments 10, 20 of the assembly 1 according to the invention that is at least similar as the user experience regarding the operation of conventional laparoscopic invasive instruments.

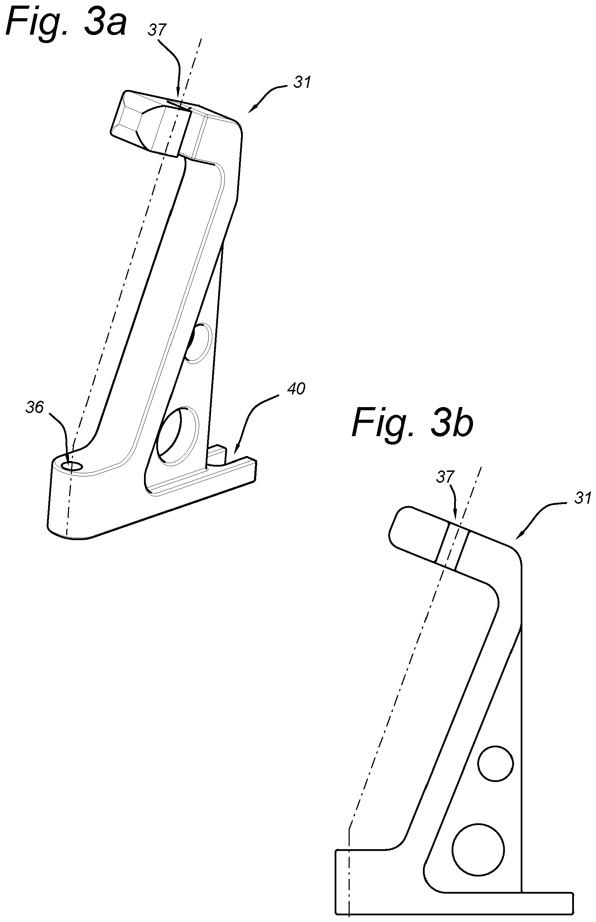

Figures 1a-1d show that the same kind of bracket 31 of the assembly 1 according to the invention is used for both steerable instruments 10, 20. As shown in figures 1a-1d, the bracket 31 is used up-side down for one of the steerable instruments 10, 20. An advantage of this approach is that no specific brackets for each of the instruments 10, 20 need to be provided. The skilled person will appreciate that other exemplary embodiments of the bracket 31 can be envisaged within the scope of the invention that can be used for both steerable instruments 10, 20 while not having to be used up-side down for one of the instruments as is required in the case of the exemplary embodiments of the brackets 31 shown in figures 1a-1d and 3a-3f.

Figure 3a shows a schematic perspective view of a non-limiting embodiment of a bracket 31 that can be used for setting one triangulation angle. Figure 3b shows a schematic side view of the bracket 31 shown in figure 3a. Bracket 31 as shown in figures 3a and 3b comprises a first receiving means 36, which in this non-limiting embodiment is a channel. The first receiving means 36 is adapted for receiving the rigid intermediate part 12 of a steerable instrument 10. Bracket 31 furthermore comprises a second receiving means 37, which in this non-limiting embodiment is a slit. The slit is adapted for proving a releasable coupling with a part of the proximal end part 11 that is located between the first and second proximal flexible zones 14, 15 of a steerable instrument 10. It is noted, that despite the engagement of the bracket 31 and the steerable instrument 10, the steerable instrument 10 preferably is rotatable around its own longitudinal axis.

It will be clear to the skilled person that other embodiments of the first and/or second receiving means such as clamps can be envisaged which fall within the scope of the invention. However, it is noted, that despite the engagement of the bracket 31 and the steerable instrument 10, the steerable instrument 10 preferably is rotatable around its own longitudinal axis.

As can be seen in figures 1a-1d, preferably the proximal flexible zone 14 is arranged between the channel 36 and the slit 37. The triangulation angle is preset and can have any value in a range of 10°- 45°. When using a bracket 31 according to the exemplary embodiment as shown in figures 3a and 3b, it would be required to have access to a set of brackets 31 each having a different preset triangulation angle in the case that the triangulation angle would need to be changed from one value to another. In certain situations this might be disadvantageous.

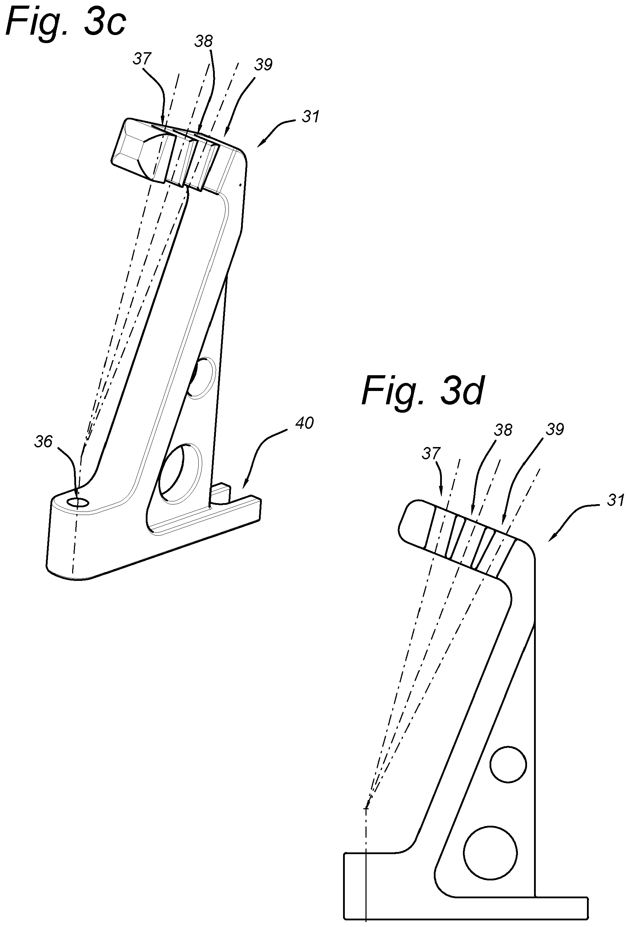

Figure 3c shows a schematic perspective view of another non-limiting embodiment of a bracket 31 that can mitigate the disadvantage of the bracket 31 as shown in figures 3a and 3b. Figure 3d shows a schematic side view of the bracket 31 shown in figure 3c. The bracket 31 as shown in figures 3c and 3d comprises three slits 37, 38, 39 that each are adapted for establishing a releasable coupling with a part of the proximal end part 11 that is located between the first and second proximal flexible zones 14, 15 at a different angle with respect to the rigid intermediate part 12 of a steerable instrument 10. Preferably, the proximal flexible zone 14 is positioned between the channel 36 and one of the slits 37, 38, 39. The values of the triangulation angle that can be set by each of the slits 37, 38, 39 are preset and can have any value in a range of 10° - 45°, for example 10° for slit 37, 25° for slit 38, and 40° for slit 39. However, it will be clear to the skilled person that other values can be envisaged, for example 20° for slit 37, 30° for slit 38, and 40° for slit 39.

In the non-limiting embodiment of the assembly 1 according to the invention as shown in figures 1a-1d, the assembly 1 further comprises a rotation setting arrangement that is adapted for setting a rotation of the invasive instrument 10 about a rotation axis extending in the guiding direction of the guiding arrangement, i.e. in the guiding direction of the rigid shaft 33. An advantage of the assembly 1 according to the invention is that the rotation setting arrangement allows setting of different working planes of the distal end parts 13, 23 of the steerable instruments 10, 20 and the associated tools 2 for each of the instruments individually. In this way, the rotation setting arrangement enables controllability of another important degree of freedom of the movement of the steerable instrument 10, 20 and the associated tool 2 at its distal end.

Figures 1a-1d show a non-limiting embodiment of the rotation setting arrangement of the access device 30, wherein the rotation setting arrangement comprises a pin 50, and a frame 31 that has one recess 40 for engaging with the pin 50. According to this non-limiting embodiment of the rotation setting arrangement, the bracket 31 of the deflection setting arrangement is used for the frame. In this way, a part of the rotation setting arrangement and the deflection setting arrangement can be integrated in a single component, i.e. bracket 31. As a result, two degrees of freedom of the movement of a steerable instrument 10 and the associated tool 2 at its distal end 13 can be controlled by the single bracket 31 according to the invention.

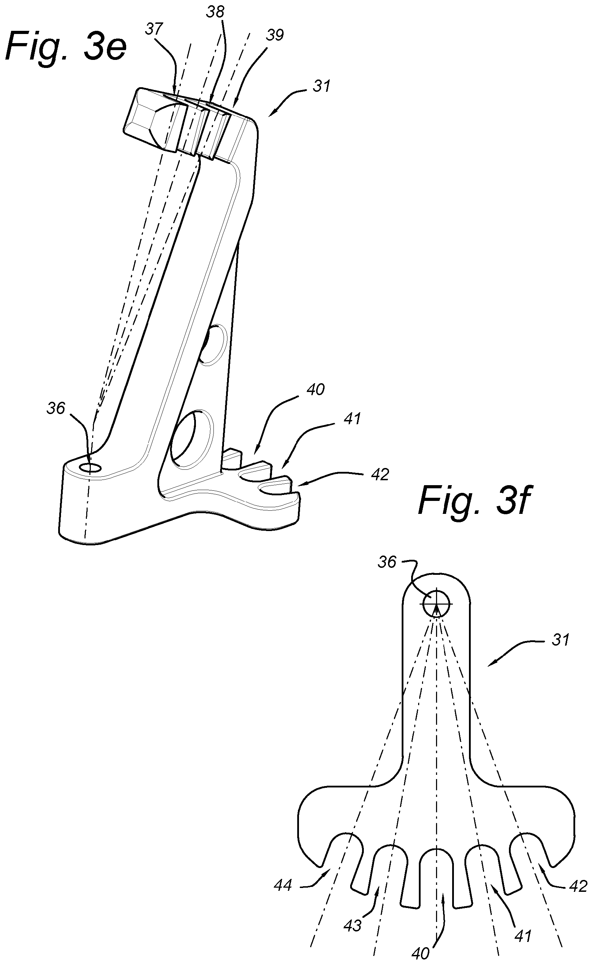

Figure 3e shows a schematic perspective view of a non-limiting embodiment of the bracket 31 comprising three second receiving means, i.e. slits 37, 38, and 39, for setting different preset triangulation angles as described above, and five third receiving means 40, 41, 42, 43, and 44, that each are adapted for engaging with the pin 50 of the rotation setting arrangement. The skilled person will appreciate that the bracket 31 can comprise any required number of both the second and third receiving means.

Figure 3f shows a bottom view of the embodiment of the bracket 31 shown in figure 3e. In the non-limiting embodiment of the bracket 31 as shown in figure 3e, the third receiving means 40, 41, 42, 43, and 44 are recesses that are adapted for proving a releasable coupling with the pin 50. The recesses 40, 41, 42, 43, and 44 are arranged according to a periphery of an imaginary cylinder that is coaxially arranged with the rigid intermediate part 12 of the steerable instrument 10. In this way, the different recesses 40, 41, 42, 43, and 44 allow different rotation angles to be set upon engaging with the pin 50. It will be clear to the skilled person that other embodiments of the third receiving means such as clamps can be envisaged which fall within the scope of the invention.

The five recesses 40, 41, 42, 43, and 44 of the bracket 31 as shown in figures 3e and 3f each provide a preset rotation angle that can have any value in a range of -45°-+45°, for example -30° for slit 42, -15° for slit 41, 0° for slit 40, +15° for slit 43, and +30° for slit 44. However, it will be clear to the skilled person that other values can be envisaged.

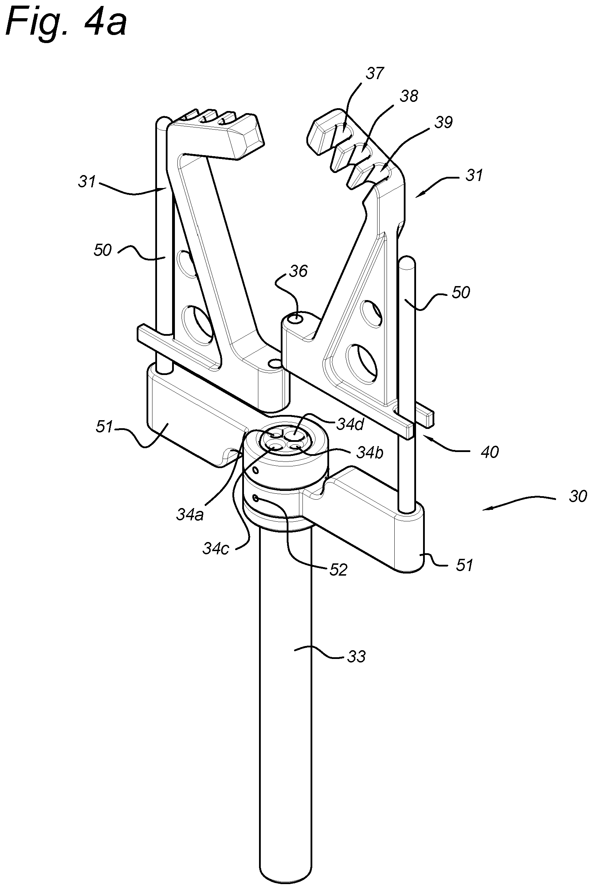

Figure 4a shows a schematic perspective view of another non-limiting embodiment of the rotation setting arrangement of the access device 30 according to the invention. According to this embodiment the pin 50 is pivotably connected to the guiding arrangement 33 via an arm 51 that is arranged to allow the pin 50 and the bracket 31, which are engaged via the recess 40, to be movable according to a periphery of an imaginary cylinder having a longitudinal axis that extends parallel to a longitudinal axis of the rigid intermediate part 12 of the instrument 10. An advantage of the embodiment of the rotation setting arrangement as shown in figure 4a is that the rotation angle can be set by rotation of the arm 51. Hence, it is not required to provide the bracket 31 with multiple third receiving means, one of which would have to be selected for setting a specific rotation angle. Consequently, the bracket 31 would only need to have one third receiving means, i.e. recess 40 as is shown in figure 4a. However, for redundancy reasons the bracket 31 could nevertheless be provided with more than one recess, e.g. with five recesses 40, 41, 42, 43, and 44 according to the non-limiting embodiment of bracket 31 as shown in figures 3e and 3f.

According to the exemplary embodiment of the rotation setting arrangement as shown in figure 4a, the movement of the arm 51 according to a periphery of an imaginary cylinder as described above, can be restricted by a fastening means 52, such as a socket screw. The skilled person will know of appropriate fastening means that can be used for this purpose.

Furthermore, the skilled person will appreciate that other embodiments of the rotation setting arrangement can be envisaged falling within the scope of this invention. One such exemplary embodiment (not shown) could be a rotation setting arrangement wherein the arm comprises a first part and a second part. The first and second parts of the arm can be pivotably coupled to each other, for example via a hinge. The first part of arm is for example connected with the pin, whereas the second part of arm is connected with the guiding arrangement. An appropriate rotation angle could be set by displacing the first part of arm with respect to the second part of arm such that the first part of arm moves according to a periphery of an imaginary cylinder having a longitudinal axis that extends parallel to a longitudinal axis of the rigid intermediate part of the instrument.

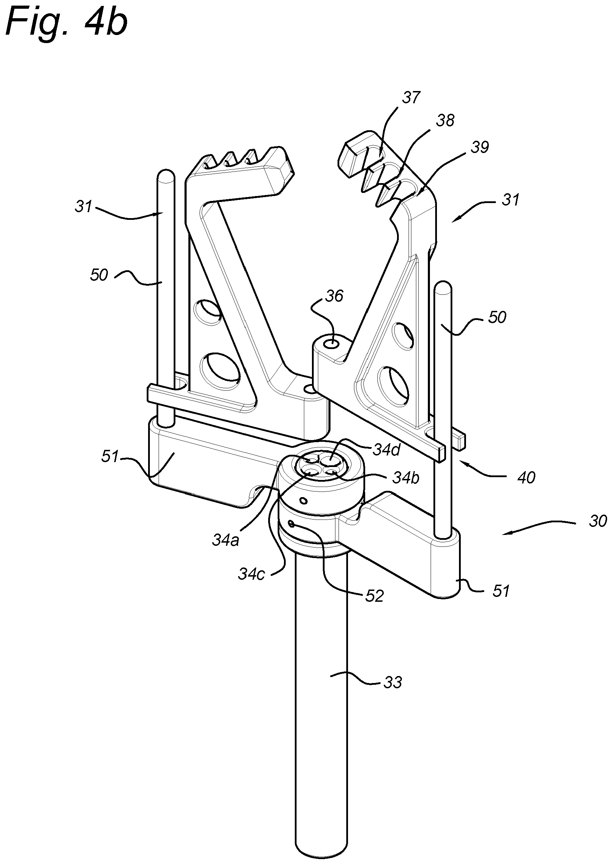

Figure 4b shows a schematic perspective view of the embodiment of the rotation setting arrangement of the access device 30 according to the invention as shown in figure 4a, wherein the arms 51 are in different positions with respect to each other thereby establishing different rotation settings as described above.

The assembly 1 according to the invention further comprises a position setting arrangement that is adapted for setting a position of the invasive instruments 10, 20 relative to the access device 30 in the guiding direction of the guiding arrangement, i.e. in the guiding direction of the rigid shaft 33. Figures 1a-1d show a non-limiting embodiment of the position setting arrangement of the assembly 1 according to the invention, wherein the position setting arrangement comprises the pin 50 of the rotation setting arrangement as described above. Moreover, the pin 50 extends in the guiding direction of the rigid shaft 33. In this way, the pin 50 can also be used to guide the instrument 10, which is connected with the pin 50 via a third receiving means of the bracket 31, in the guiding direction of the rigid shaft 33. The position of the steerable instruments 10, 20 relative to the access device 30 can be fixed by any suitable fixation means known to the skilled person.

An advantage of the position setting arrangement of the assembly 1 according to the invention as shown in figures 1a-1d and figures 4a and 4b, is that it allows setting of the positions of the invasive instruments 10, 20 relative to the access device 30 for each of the instruments 10, 20 individually as is shown in figures 1a-1d.

Based on the above, it is clear to the skilled person that the position setting arrangement according to the invention enables controllability of an important degree of freedom of the movement of the steerable instrument 10, 20 and the associated tool 2 at its distal end 13, 23 in an easy way. Furthermore, the skilled person will appreciate that the assembly 1 according to the invention allows to combine the arrangements for setting the position of the invasive instruments relative to the access device, the triangulation angle of the distal end parts of these instruments, and the rotation of the steerable instruments. As a result, at least three degrees of freedom of the movement of the steerable invasive instruments can be controlled in a very convenient and uncomplicated way.

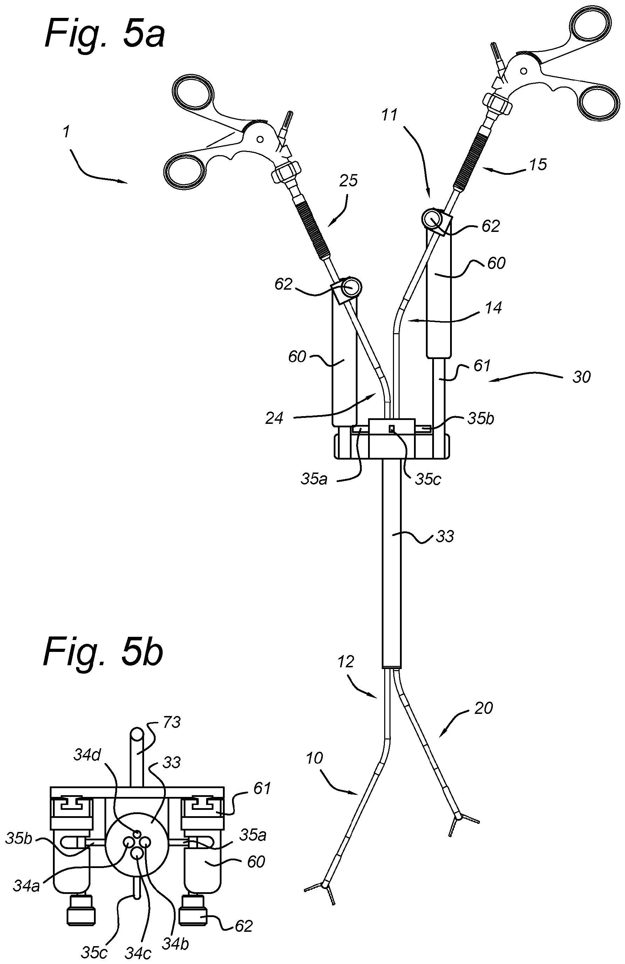

Figure 5a shows another non-limiting embodiment of the position setting arrangement of the assembly 1 according to the invention, wherein the position setting arrangement comprises a sliding comprises arms 60, 61 that are arranged to be slidable with respect to each other in order to allow the instrument 10 to be positioned relative to the access device 30. As mentioned above, an advantage of the position setting arrangement of the assembly 1 according to the invention is that it allows setting of the positions of the invasive instruments 10, 20 relative to the access device 30 for each of the instruments 10, 20 individually as is shown in figure 5a.

Figure 5a further shows another non-limiting embodiment of the deflection setting arrangement of the assembly 1 according to the invention, wherein the deflection setting means comprises an engagement means 62 that is adapted for engaging with a part of the proximal end part 11 that is located between the first and second proximal flexible zones 14, 15 of a steerable instrument 10. It is noted, that the engagement means 62 preferably is adapted to allow rotation of the steerable instrument 10 around its own longitudinal axis while it is engaged with the engagement means 62.

It will be appreciated that the skilled person will know of suitable embodiments for the engagement means such as an arrangement 62 that comprises a tubular guiding shaft and a fastening knob for fastening the arrangement 62 to arm 60 of the sliding means as is shown in figure 5a. By changing the position of the tubular guiding shaft 62 around a center line of the fastening knob, it is possible to set different values of the triangulation angle.

Figure 5b shows a cross-sectional view of the embodiment of the access device 30 shown in figure 5a. Figure 5b illustrates the implementation of the arms 60, 61 of the sliding means.

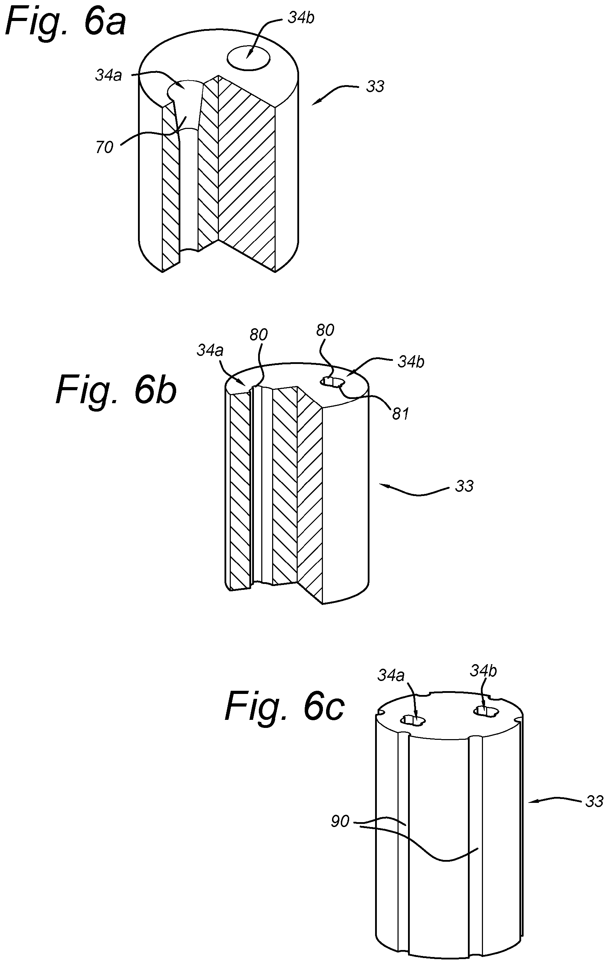

Figure 6a shows a combination of a schematic perspective view and a longitudinal cross-sectional view of a non-limiting embodiment of a part of the rigid shaft 33 of the access device 30 according to the invention. As shown in figure 6a, the rigid shaft 33 comprises two lumen 34a, 34b. The combined view shows the tapered shape 70 of the proximal end part of the lumen 34a.

An advantage of the tapered shape 70 of at least one of the lumen 34a and 34b is that an instrument with a tool, such as a forceps or a pair of scissors, arranged at its distal end part can be inserted easier into the lumen. For example, the jaw of a forceps can be in a slightly opened position that can complicate insertion of the forceps into the lumen as the jaw first needs to be forced into a closed position as a result of an interaction between the forceps and the wall surrounding the access port of the lumen before the forceps and the instrument can be inserted into the lumen. Especially in situations in which speed of action is critical, an impeded insertion of an invasive instrument is a considerable disadvantage. Loss of valuable time can pose a threat to the human or animal under surgery.

Furthermore, if fragile tools are being used, an impeded insertion could damage such tools. The tapered shape 70 of at least one of the lumen 34a and 34b can mitigate or at least reduce the abovementioned disadvantages.

It will be appreciated by the skilled person that in addition to the proximal end part of a lumen, the distal end part of a lumen can have a tapered shape 70. The tapered shape 70 of the lumen at the distal end part thereof could be beneficial for the insertion of a tool into the lumen upon retraction of the invasive instrument.

Figure 6b shows a combination of a schematic perspective view and a longitudinal cross-sectional view of another non-limiting embodiment of a part of the rigid shaft 33 of the access device 30 according to the invention. As shown in figure 6b, the rigid shaft 33 comprises two lumen 34a, 34b having two elongated recesses 80, 81that extend along the length of the lumen. It can happen that tools even after having been brought into a position in which they have minimal dimensions, extend outside the inner diameter of the lumen. By providing some additional space in a radial direction of the lumen, the two elongated recesses 80, 81 extending along the length of the lumen enable that these tools nevertheless can be inserted and passed through the lumen. It will be clear to the skilled person that the number and disposition of the elongated recesses along the circumference of the lumen can be adapted to the shape of the specific tools that are required for a particular intervention.

Figure 6c shows a schematic perspective view of yet another non-limiting embodiment of a part of the rigid shaft 33 of the access device 30 according to the invention. Figure 6c shows as a non-limiting example six elongated recesses 90 that extend along the length of the part of the rigid shaft 33. The skilled person will appreciate that depending on the requirements of the intervention the elongated recessed 90 can extend only along a part of the length of the shaft 33. Furthermore, the number and disposition of the elongated recesses 90 along the circumference of the rigid shaft 33 can be adapted to the specific requirements of a particular intervention.

The outer diameter of the shaft 33 is preferably adapted to the inner diameter of a standard trocar (not shown) to establish a snug fit when they are combined for use in a surgical intervention. However, in order to create sufficient space around the target area where the surgical intervention is to take place, a body cavity is usually being inflated using a gas such as carbon dioxide (CO₂). This gas is usually being fed into the body cavity via a clearance between the outer wall of the shaft of a conventional access device and the inner wall of a conventional trocar. A disadvantage of this approach is that a considerable clearance, in the order of several tenths of millimeters, is required. The elongated recesses 90 shown in figure 6c at least reduce the latter disadvantage as they enable a considerably smaller clearance between the outer wall of the rigid shaft 33 of the access device 30 according to the invention and the inner wall of a conventional trocar. In this way, the cross-section of the shaft can be increased which is beneficial for the diameter of the at least first lumen 34a, 34b, 34c, 34d which can also further be increased.

Furthermore, the elongated recesses 90 provide a means for a surgeon to notice any insufflations loss, e.g. audibly and/or visually. In the event that the shaft 33 of the access device 30 is retracted too far out of the trocar, the insufflation gas can flow out of the body cavity via at least a first recess 90 that acts as a leakage path for the insufflations gas. The surgeon or any other person for example could hear that the insufflation gas leaks out of the body cavity. By moving the shaft 33 further into the trocar, the insufflation loss can be eliminated or at least be reduced.

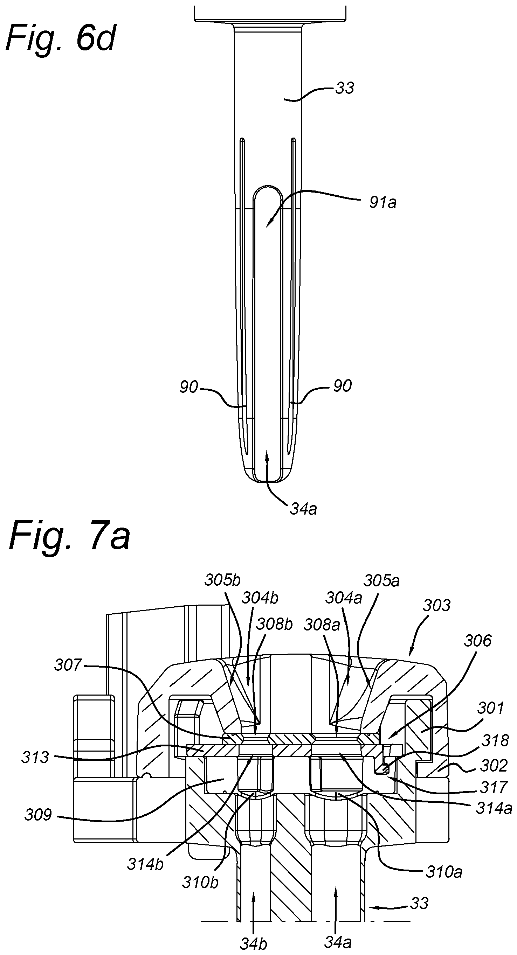

Figure 6d shows a schematic side view of another non-limiting embodiment of the rigid shaft 33 of the access device according to the invention, the shaft 33 comprising a non-limiting embodiment of the recess 90 described above. Furthermore, figure 6d shows a non-limiting embodiment of the distal end part of at least the lumen 34a that is provided with an increased aperture due to an arrangement of an elongated slot 91a in the outer wall of the shaft 33 in an axial direction of the lumen 34a. So, along a predetermined length at the distal end part of the shaft at least one of the lumens 34a, 34b, 34c, 34d is open at the outer wall. An advantage of the embodiment of the distal end part of at least the lumen 34a as shown in figure 6d, is that the increased aperture at the distal end part of the lumen 34a facilitates improved cleaning of the shaft 33 as the increased aperture enables easier access to the lumen 34a for fluidic and mechanical cleaning means. Another advantage of this embodiment is that the manufacturability of the shaft 33 is enhanced due to easier material removal via the increased aperture of the lumen during manufacturing of the lumen. It will be appreciated that this embodiment of the distal end part of the lumen can be applied to one, a selection of or all lumen that are applied in the shaft 33 according to the requirements for the access device.

In the exemplary embodiment of the shaft 33 schematically shown in figure 6d, the elongated recesses 90 extend further in an axial direction of the shaft 33 towards the proximal end part of the shaft than the elongated slot 91a that provides an increased aperture to lumen 34a. It will be appreciated that in another embodiment of the shaft 33, the at least one elongated recess can be arranged to extend equally far in an axial direction of the shaft 33 towards the proximal end part of the shaft as the elongated slot 91a. In the latter case, insufflation loss will be detected at a later stage , i.e. when the shaft 33 is retracted further out of the trocar as the at least one elongated recess has to pass the seal of the trocar that is arranged to enclose at least a part of the outer wall of the shaft 33.

Figure 6d schematically shows that an elongated recess 90 and an elongated slot 91a are arranged adjacent to each other in a circumferential direction of the shaft 33.

It is observed that the arrangement with one or more open lumens 34a, 34b, 34c, 34d and one or more elongated recesses 90 can be used in any access device that is adapted for introducing at least one invasive instrument into an object via an access port of the object. So, the invention also applies to an access device comprising a shaft with at least one lumen each arranged to receive an elongated instrument, the shaft having an outer wall which is provided with at least one elongated slot that is arranged in an axial direction of said at least one lumen to provide an increased aperture of said at least one lumen at a distal end part of said lumen, as well as at least one elongated recess arranged adjacent to said at least one elongated slot in a circumferential direction of the shaft.

Figure 7a shows a schematic cross-sectional view of a non-limiting embodiment of the proximal end of the shaft 33 that is connected with a capping element 303 and a sealing arrangement 306 according to an embodiment of the invention. From figure 7a it can be observed that the proximal end of the shaft 33 comprises a first element 301 of a coupling arrangement, which first element 301 is, preferably, an integral part of said shaft 33. The capping element 303 comprises a second element 302 of the coupling arrangement. The second element 302 can be connected with the first element 301 for connecting the capping element 303 with the proximal end of the shaft 33. In the non-limiting embodiment of the coupling arrangement shown in figure 7a, the first 301 and second 302 elements are arranged to provide a bayonet coupling. According to another exemplary embodiment of the coupling arrangement, the first 301 and second 302 elements can be arranged to provide a click-on coupling. However, any other coupling / decoupling arrangement known to persons skilled in the art can be applied as well.

Figure 7a schematically shows that the capping element 303 is provided with a first set of through holes comprising at least a first through hole 304a and a further through hole 304b that are both arranged to provide respective passages between a proximal end face and a distal end face of the capping element 303. The at least first through hole 304a and the further through hole 304b are arranged to enable insertion of an invasive instrument into at least one of lumen 34a and lumen 34b, respectively.

Figure 7a shows that the at least first through hole 304a and the further through hole 304b at least partly have tapered shapes 305a, 305b that are configured to narrow down towards the distal end face of the capping element 303. As can be seen from figure 7a, the tapered shape 305a, 305b of the at least first through hole 304a and the further through hole 304b have wider diameters at the proximal end face of the capping element 303 than at the distal end face of the capping element 303. As a result of the at least partly tapered shape 305a, 305b of the at least first through hole 304a and the further through hole 304b, insertion of an invasive instrument is improved.

Furthermore, figure 7a shows that the access device according to the invention further comprises at least a first sealing arrangement 306 that is adapted for sealing at least lumen 34a and lumen 34b of the shaft 33 in a fluid-tight manner. In the non-limiting embodiment shown in figure 7a, the at least first sealing arrangement 306 is connected with the proximal end face of the shaft 33. The sealing arrangement 306 has to seal at least lumen 34a and lumen 34b of the shaft 33 in a fluid-tight manner under all circumstances, i.e. in a first situation in which an invasive instrument, steerable or rigid, is inserted in at least one of lumen 34a and lumen 34b of the shaft 33, and in a second situation in which no invasive instrument is inserted in either one of lumen 34a and lumen 34b. It is remarked that in the context of the present application the term rigid invasive instrument is to be construed as a non-steerable invasive instrument unless otherwise indicated.

Moreover, according to the embodiment shown in figure 7a, the at least first sealing arrangement 306 comprises a first member 307 comprising a resilient material that is provided with a second set of through holes comprising at least a second through hole 308a and a further through hole 308b that are arranged to enable insertion of an invasive instrument into at least one of lumen 34a and lumen 34b, respectively. The at least second through hole 308a is arranged to define an inner surface of the resilient material surrounding the at least second through hole 308a. The inner surface of the resilient material is adapted for enclosing at least a part of an outer surface of the proximal end part of an invasive instrument to provide a fluid-tight seal for lumen 34a of the shaft 33. The same applies to the further through hole 308b of the second set of through holes with respect to providing a fluid-tight seal for lumen 34b of the shaft 33.

In the context of the present application, a resilient material suitable for application in the first member 307 of the sealing arrangement 306 is to be construed as a material that is capable of coming into and remaining in abutting contact with an outer surface of an invasive instrument that is inserted through a through hole provided in the resilient material. In this way, the resilient material enables a fluid-tight seal of a respective lumen of the shaft 33. Examples of suitable resilient materials are silicones or elastomers, e.g. thermoplastic elastomers (TPE) like TPO, TPU, TPS, etc .

Figure 7a schematically shows that the at least second through hole 308a and the further through hole 308b have respective diameters that are smaller than respective smallest diameters of lumen 34a and lumen 34b of the shaft 33 with which they are aligned respectively. Furthermore, in a non-limiting embodiment according to the invention, the first member 307 of the sealing arrangement 306 is disc-shaped. It will be appreciated that the number of though holes of the second set of through holes provided in the first member 307 of the sealing arrangement 306 can be chosen according to the requirements for the shaft 33 of the access device. However, in practice, the number of through holes of the second set of through holes preferably is equal to the number of through holes of the first set of through holes provided in the capping element 303 and to the number of lumen of the shaft 33 of the access device.

Furthermore, the non-limiting embodiment of figure 7a schematically shows that the sealing arrangement 306 further comprises a second member 309 that is connectable with the first member 307 via a third member 313. The second member 309 comprises a resilient material that is provided with a first set of valves comprising at least a first valve 310a that is arranged to enable insertion of an invasive instrument into lumen 34a when the at least first valve 310a is in a first or at least partly opened position and to provide a fluid-tight seal for lumen 34a when the at least first valve 310a is in a second position or closed position, in which latter position no invasive instrument is inserted through the at least first valve 310a. The same applies to the further valve 310b of the first set of valves with respect to enabling insertion of an invasive instrument into lumen 34b when the further valve 310b is in a first or at least partly opened position and to providing a fluid-tight seal for lumen 34b when the further valve 310b is in a second or closed position.

In the context of the present application, a resilient material suitable for application in the second member 309 of the sealing arrangement 306 is to be construed as a material that is capable of maintaining its shape when an invasive instrument is inserted through a valve of the first set of valves. Examples of suitable resilient materials for fabricating the second member 309 and the valves are silicones or elastomers, e.g. thermoplastic elastomers (TPE) like TPO, TPU, TPS, etc. It will be appreciated that the resilient material for the second member 309 could be different from the resilient material for the valves of the second member.

It will be appreciated that the number of valves of the first set of valves provided in the second resilient member 309 of the sealing arrangement 306 can be chosen according to the requirements for the shaft 33 of the access device. However, in practice, the number of valves of the first set of valves preferably is equal to the number of through holes of the second set of through holes provided in the first resilient member 307 of the sealing arrangement 306 as well as to the number of through holes of the first set of through holes provided in the capping element 303 and to the number of lumen of the shaft 33 of the access device 30.

Furthermore, the non-limiting embodiment of figure 7a schematically shows that the at least first sealing arrangement 306 further comprises a third member 313 that is arranged between the first member 307 and the second member 309. The third member 313 comprises a rigid material. Examples of suitable rigid materials for fabricating the third member 313 are polymers, e.g. thermoplastic polymers like acrylonitrile butadiene styrene (ABS) and polycarbonate (PC).

The third rigid member 313 is provided with a third set of through holes comprising at least a third through hole 314a that is arranged to enable insertion of an invasive instrument into lumen 34a of the shaft 33. The further through hole 314b of the third set of through holes is also arranged to enable insertion of an invasive instrument into lumen 34b of the shaft 33. Furthermore, in a non-limiting embodiment according to the invention, the third member 313 of the sealing arrangement 306 is disc-shaped.

Furthermore, it can be seen that according to the non-limiting embodiment as schematically shown in figure 7a, the sealing arrangement 306 is arranged between the capping element 303 and the proximal end face of the shaft 33. In the assembled state, i.e. when the first element 301 and second element 302 are connected with one another, the second element 302 clamps the sealing arrangement 306 against the proximal end face of the shaft 33 in a fluid-tight manner. The second resilient member 309 is arranged into a suitable opening provided by the first element 301 such that, in use, the valves 310a and 310b of the first set of valves are aligned with the lumen 34a and 34b respectively. The third rigid member 313 is arranged on top of and fixed to the second resilient member 309 such that when, in use, a first elongated invasive instrument is inserted into a first lumen of the shaft 33, the third member 313 takes care that the resilient second member 309 is kept in place such that each one of the valves of the second resilient member 309 remains in an aligned arrangement with a respective through hole of the third member 313 and a respective lumen of the shaft 33 of the access device 30. For fixing the third rigid member 313 and the second resilient member 309 to one another, preferably, the second resilient member 309 is provided with at least one recess 317 and the third rigid member 313 comprises at least one protrusion 318, wherein the at least one recess 317 of the second resilient member 309 is configured to accommodate the at least one protrusion 318 of the third member 313 to form a locked state. The at least one protrusion 318 of the third rigid member 313 and the at least one recess 317 of the second resilient member 309 can be arranged to form one of a bayonet lock, a snap-fit lock and a click-on lock. It will be appreciated that alternatively the third rigid member 313 can be provided with at least one recess and that the second resilient member comprises at least one protrusion for establishing a coupling between the third 313 and second 309 members.

The first resilient member 307 is arranged on top of the third rigid member 313 such that, in use, the through holes 308a and 308b of the second set of through holes of the first resilient member 307 are aligned with respective through holes 314a and 314b in the third rigid member 313, respective valves 310a and 310b in the second resilient member 309 and with respective lumen 34a and 34b of the shaft 33 to enable insertion of an invasive instrument into at least one of the lumen 34a and 34b, respectively. The first resilient member 307 preferably is chemically connected or chemically bonded with the third rigid member 313. This can for example be done by applying a so-called two component (2k) injection moulding technique in which the third rigid member 313 comprises a first component of the two components and the resilient first member 307 comprises a second component of the two components. Examples of suitable materials for the first component are polymers, e.g. thermoplastic polymers like acrylonitrile butadiene styrene (ABS) and polycarbonate (PC). Examples of suitable materials for the second component are silicones or elastomers, e.g. thermoplastic elastomers (TPE) like TPO, TPU, TPS, etc .

Finally, the capping element 303 is arranged to engage at least with the first resilient member 307 such that, in use, the through holes 304 a and 304b of the first set of through holes of the capping element 303 are in alignment with the through holes 308a and 308b of the second set of through holes of the first resilient member 307, respective through holes 314a and 314b in the third rigid member 313, respective valves 310a and 310b in the second resilient member 309 and with respective lumen 34a and 34b of the shaft 33 to enable insertion of an invasive instrument into at least one of the lumen 34a and 34b, respectively.

The capping element 303 is connectable with the proximal end face of the shaft 33 by connecting the first 301 and second 302 elements of the coupling arrangement with one another. The coupling arrangement can be arranged to form one of a bayonet lock, a snap-fit lock and a click-on lock.

The sealing arrangement 306 can be provided as a separate component that can be used in cooperation with the capping element 303 that is arranged to keep the sealing arrangement 306 in place and aligned with the lumen of the shaft 33 to enable insertion of an invasive instrument in at least one of these lumen. However, it will be appreciated that according to another non-limiting embodiment of the invention, the capping element 303 and the sealing arrangement 306 can also be arranged to form one integrated unit, i.e., a unit in which these elements are fixed to one another. In this case, when removing the capping element 303, the sealing arrangement 306 is automatically removed.

It is also observed that the sealing arrangement shown in figure 7a can be used to seal any arbitrary shaped lumen in a shaft that should be accessible for an elongated instrument where such a seal should be fluid-tight both in the situation where the instrument is inserted into and is not inserted into the lumen. In its basic form, this part of the invention relates to a sealing arrangement comprising a first member that comprises a resilient material that is provided with a first set of valves comprising at least one valve, and at least a first element (317) of an adjustable connecting arrangement, a second member comprising a rigid material that is provided with a first set of through holes comprising at least one through hole, and at least a second element (318) of the adjustable connecting arrangement, the at least first element (317) and the at least second element (318) being configured to enable an adjustable connection of the first member and the second member to one another in a locked state, the first set of valves being arranged in alignment with the first set of through holes in the locked state.

The first 307, second 309 and third members 313 of the sealing arrangement 306 and the capping element 303 may be provided as a separate set of elements. Alternatively, they may be provided in a mutually fixed state where the third rigid member 313 is arranged on top of and fixed to the second resilient member 309, and the first resilient member 307 is arranged on top of and fixed to the third rigid member 313, and finally, the capping element 303 is arranged on top of and fixed to the first resilient member 307.

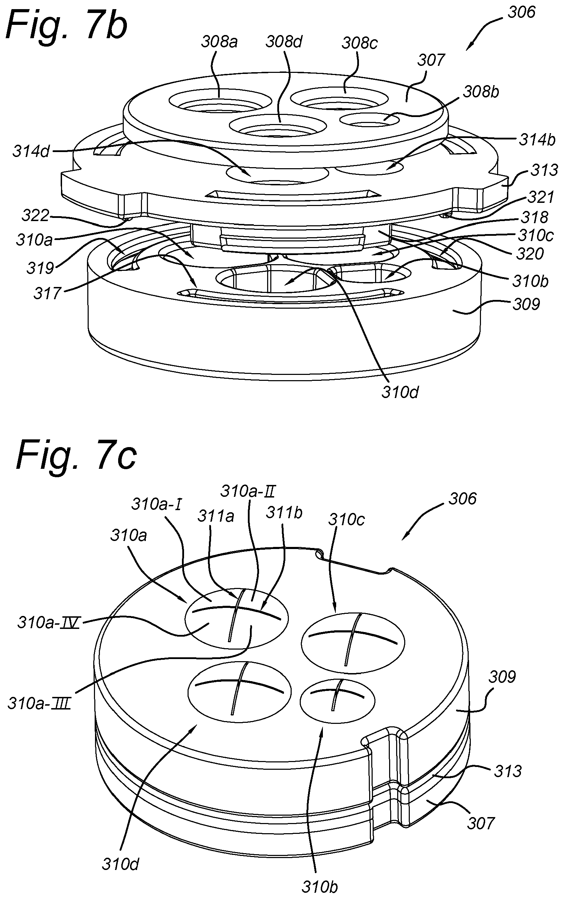

Figure 7b shows a schematic exploded view of a non-limiting embodiment of a sealing arrangement 306 according to the invention. It can clearly be seen that according to this exemplary embodiment the first member 307 of the sealing arrangement 306 is provided with a second set of through holes comprising four through holes 308a, 308b, 308c and 308d, the second member 309 of the sealing arrangement 306 is provided with a first set of valves comprising four valves 310a, 310b, 310c and 310d, and that the third member 313 of the sealing arrangement 306 is provided with a third set of through holes comprising four through holes 314a, 314b, 314c and 314d of which only through holes 314b and 314d can be seen in figure 7b.

Furthermore, figure 7b shows that the second resilient member 309 is provided with an exemplary number of three recesses 317, 319 and 320 and that the third rigid member 313 comprises three protrusions 318, 321 and 322 that are arranged to fix the second resilient member 309 and the third rigid member 313 to one another in a locked state.

Figure 7c shows a schematic perspective, upside down view of a non-limiting embodiment of a sealing arrangement 306 according to the invention and especially of a non-limiting embodiment of the second member 309 of the sealing arrangement 306. The first set of valves comprises an exemplary number of four valves 310a, 310b, 310c and 310d that each comprise a resilient material that is provided with at least two intersecting slits 311a and 311b. These intersecting slits can be arranged to define at least three valve parts. In the exemplary embodiment of the second member 309 of the sealing arrangement 306 as shown in figure 7c, the resilient material of each of the four valves 310a, 310b, 310c and 310d is provided with two intersecting slits 311a and 311b that define four valve parts for each of the valves. In the case of valve 310a, these valve parts have been indicated as 310a-I, 310a-II, 310a-III and 310a-IV, respectively.

When the valve parts of a particular valve are in a first or at least partly opened position, this particular valve enables insertion of an invasive instrument into a lumen of the shaft 33 with which that valve is in an aligned arrangement. When the valve parts of a particular valve are in a second or closed position, this particular valve provides a fluid-tight seal for the lumen of the shaft with which the valve is in an aligned arrangement. In the context of the present application, a resilient material suitable for application in any of the valves 310a, 310b, 310c and 310d of the first set of valves of the second member 309, is to be construed as a material that is capable of resuming its original shape when an invasive instrument is retracted out of a valve. Examples of suitable resilient materials for fabricating the second member 309 and the valves are silicones or elastomers, e.g. thermoplastic elastomers (TPE) like TPO, TPU, TPS, etc. It will be appreciated that the resilient material for the second member 309 could be different from the resilient material for the valves of the second member.

Furthermore, it will be appreciated that according to a non-limiting embodiment of the second member 309 of the sealing arrangement 306, the valve parts of the valves can be configured to open upon contact of at least a part of the valve with at least a part of an outer wall of an elongated body of an invasive instrument. In this way, contact between the valve parts of a valve and a tool that is arranged at the distal end part of the elongated body of the invasive instrument can preferably be prevented but at least be minimized.

Although the valves of the second member 309 preferably are passive elements, i.e. without requiring any electrical connections for operating them between a first or at least partly opened position and a second or closed position, it will be appreciated that electrically operated valves can also be implemented.

The skilled person will appreciate that a non-limiting embodiment of the second member 309 of the sealing arrangement 306 is disc-shaped.

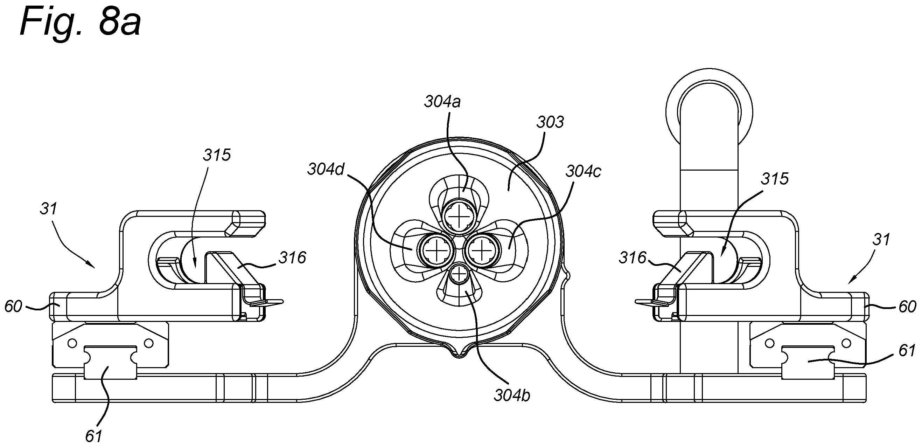

Figure 8a shows a schematic top view of non-limiting embodiments of both the capping element 303 and a bracket 31 according to the invention. It can be seen that the capping element 303 is provided with a first set of through holes comprising an exemplary number of four through holes 304a, 304b, 304c and 304d that have at least partly a tapered shape in order to facilitate improved insertion of an invasive instrument into any one of the lumen of the shaft of the access device that is in aligned arrangement with any one the through holes of the first set of through holes.

Furthermore, figure 8a schematically shows a non-limiting embodiment of a bracket 31 that comprises a first receiving space 315 that is adapted for receiving at least a part of the proximal end part of an invasive instrument. The bracket 31 comprises a retaining member 316 that is adapted for retaining the part of the proximal end part of the invasive instrument within the receiving space. The retaining member 316 preferably enables that a surgeon can insert at least a part of the proximal end part of the elongated tubular body of an invasive instrument with one hand only. In addition, the retaining member can be constructed and arranged such that removal of the proximal end part of the elongated tubular body of the invasive instrument can also be achieved by just using one hand. In a non-limiting embodiment the retaining member 316 can comprise a snap-lock element.

It will be appreciated that the bracket 31 and the retaining member 316 can be provided as separate components that can be assembled as is shown in the exemplary embodiment of the access device 30 as shown in figure 8a. However, it will be appreciated that in another non-limiting embodiment of the access device 30 according to the invention, the bracket 31 and the retaining member 316 can be arranged to form one integrated unit. This could be done by fabricating the bracket 31 and the retaining member 316 out of one piece of material, e.g. an aluminum alloy that has resilient properties. Preferably, the aluminum alloy will be coated in order to reinforce it. The bracket 31 and the retaining member 316 can be fabricated for example by turning and milling. It will be appreciated that other suitable fabrication techniques known to the skilled person can also be applied.

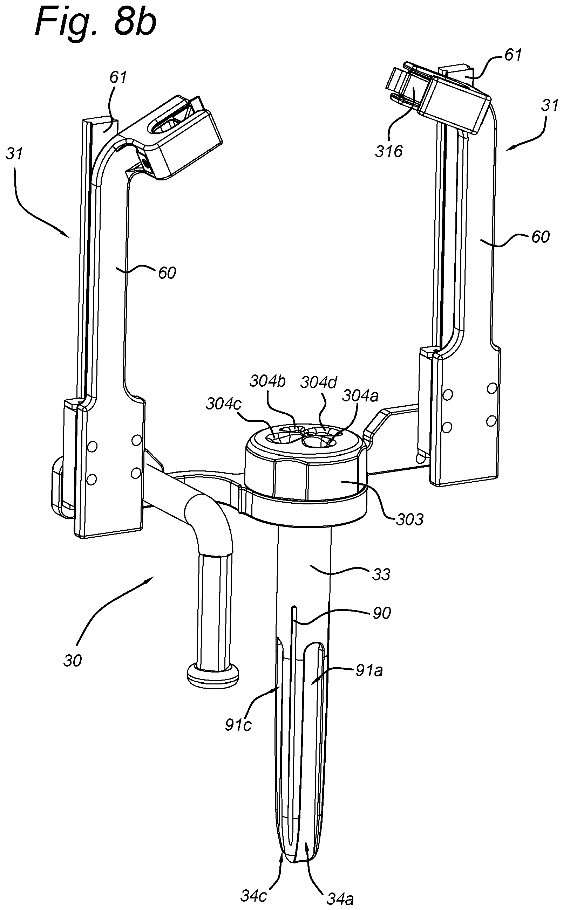

Figure 8b shows a schematic perspective view of another non-limiting embodiment of the access device 30 according to the invention. It can be seen that in this embodiment the proximal end of the shaft 33 is connected with capping element 303 that is provided with a first set of four through holes 304a, 304b, 304c and 304d that are in an aligned arrangement with the lumen of the shaft 33.

Furthermore, it can be observed that the shaft 33 is provided with at least one elongated recess 90 that can act as a leakage path for insufflation gas if the shaft 33 of the access device 30 is retracted too far out of a trocar, i.e. past a sealing member of the trocar that established a fluid-tight seal between the trocar and the shaft 33 of the access device 30.

Figure 8b also schematically shows the brackets 31 of the access device 30. Each one of the brackets 31 comprises a receiving space 315 that is adapted for receiving at least a part of the proximal end part of an invasive instrument. Each one of the brackets 31 also comprises a retaining member 316 that is adapted for retaining the part of the proximal end part of the invasive instrument within the receiving space.

It will be clear to a person skilled in the art that the scope of the invention is not limited to the examples discussed in the foregoing, but that several amendments and modifications thereof are possible without deviating from the scope of the invention as defined in the attached claims. While the invention has been illustrated and described in detail in the figures and the description, such illustration and description are to be considered illustrative or exemplary only, and not restrictive. The present invention is not limited to the disclosed embodiments but comprises any combination of the disclosed embodiments that can come to an advantage.

In the description and claims, the word "comprising" does not exclude other elements, and the indefinite article "a" or "an" does not exclude a plurality. In fact it is to be construed as meaning "at least one". Any reference signs in the claims should not be construed as limiting the scope of the invention.

## Claims

1. An access device (30) that is adapted for guiding at least one steerable invasive instrument (10, 20) into and out of an object via an access port of the object, the at least one steerable invasive instrument comprising
an elongated tubular body (18) having a proximal end part (11, 21) comprising a proximal zone (158) and at least one proximal flexible zone (156) located distal to said proximal zone (158), a distal end part (13, 23) comprising at least one distal flexible zone (154) and a rigid intermediate part (155), wherein the steerable invasive instrument further comprises a steering arrangement that is adapted for translating a deflection of the at least one proximal flexible zone (156) relative to the rigid intermediate part (155) into a related deflection of said at least one distal flexible zone (154);
wherein the access device (30) comprises
a rigid shaft (33) extending in an axial direction and having at least one lumen (34a, 34b, 34c, 34d) whereby the at least one lumen is arranged to provide a passage between a proximal end face and a distal end face of the shaft (33) and is adapted for receiving and guiding the at least one steerable invasive instrument into and out of the object in a direction parallel to said axial direction of the rigid shaft (33); and **characterized by**
a position setting arrangement and a deflection setting arrangement comprising either:
a pin (50) extending in a direction parallel to said axial direction of said rigid shaft (33) wherein said pin (50) is connected to said rigid shaft (33) via a first arm (51) and a bracket (31) comprising a recess (40) for engaging with said pin (50), a channel (36) to receive said rigid intermediate part (12) and a slit (37) to releasably couple said proximal zone (158) of said steerable invasive instrument, wherein said proximal flexible zone (156) is arranged between said channel (36) and said slit (37), such that when said at least one steerable invasive instrument is inserted into said at least one lumen and said proximal zone (158) is coupled by said bracket (31) a position of the at least one steerable invasive instrument can be set relative to said rigid shaft (33) in a direction parallel to said axial direction and such that an angle is set between said at least one proximal flexible zone (156) and said rigid intermediate part (155), and thus setting a triangulation angle between said at least one distal flexible zone (154) and said rigid intermediate part (155) of the at least one steerable invasive instrument (10, 20); or
a second arm (61) that is connected to the shaft (33) and a third arm (60) which is slidably arranged with respect to said second arm (61), said second arm (61) extending in a direction parallel to said axial direction, said third arm (60) being provided with an engagement means (62; 315, 316) adapted for engaging said proximal zone (158) of said steerable invasive instrument, such that when said at least one steerable instrument is inserted into said at least one lumen and said proximal zone (158) is engaged by said engagement means (62; 315, 316) a position of the at least one steerable invasive instrument can be set relative to the shaft in a direction parallel to said axial direction and such that an angle is set between said at least one proximal flexible zone (156) and the rigid intermediate part (155), and thus setting a triangulation angle between said at least one distal flexible zone (154) and said rigid intermediate part (155) of the at least one steerable invasive instrument (10, 20).

2. The access device (30) according to claim 1,
said pin (50) and bracket (31) being pivotably connected to the shaft (33) via the first arm (51) such that a rotation of the steerable invasive instrument (10, 20) can be set about a rotation axis extending in the axial direction of the shaft (33).

3. The access device (30) according to claim 1, wherein said bracket (31) has a plurality of receiving means (40, 41, 42, 43, 44) for engaging with the pin (50).

4. The access device (30) according to claim 1, wherein a wall of the at least one lumen is provided with at least one elongated recess (80, 81) extending along the length of the at least one lumen.

5. The access device (30) according to claim 1, wherein an outer wall of the shaft (33) is provided with at least one elongated recess (90) extending along at least a part of the length of the shaft.

6. The access device (30) according to claim 1, further comprising
a coupling arrangement that comprises a first coupling element (301) and a second coupling element (302), wherein said first coupling element (301) is arranged at a proximal end of the shaft (33);
a capping element (303) that comprises said second coupling element (302) of said coupling arrangement, said second coupling element (302) being connectable with said first coupling element (301) for connecting said capping element (303) with the proximal end of the shaft (33), said capping element (303) being provided with a first plurality of through holes (304a, 304b, 304c, 304d) comprising at least a first through hole that is arranged to provide a passage between a proximal end face and a distal end face of the capping element (303), said at least first through hole being arranged to enable guiding of said at least one invasive instrument (10, 20, 240) into and out of said at least one lumen (34a, 34b, 34c, 34d).

7. The access device according to claim 6, further comprising at least a first sealing arrangement (306) that is arranged between the capping element (303) and the proximal end face of the shaft (33) and is adapted for sealing said at least one lumen (34a, 34b, 34c, 34d) of the shaft (33) in a fluid-tight manner.

8. The access device according to claim 7, said first sealing arrangement (306) comprising an adjustable connecting arrangement; and
a first sealing member (309) that comprises a resilient material that is provided with a plurality of valves (310a, 310b, 310c, 310d) and with at least a first connection element (317) of said adjustable connecting arrangement;
a second sealing member (313) comprising a rigid material that is provided with a second plurality of through holes (314a, 314b, 314c, 314d) and with at least a second connection element (318) of said adjustable connecting arrangement, said at least first connection element (317) and said at least second connection element (318) being configured to enable an adjustable connection of said first sealing member (309) and said second sealing member (313) to one another in a locked state, said plurality of valves (310a, 310b, 310c, 310d) being arranged in alignment with said second plurality of through holes (314a, 314b, 314c, 314d) in said locked state; and
a third sealing member (307) that is connected with said second sealing member (313), said third sealing member (307) comprising a resilient material that is provided with a third plurality of through holes (308a, 308b, 308c, 308d) that are arranged in alignment with said second plurality of through holes (314a, 314b, 314c, 314d) of said second sealing member (313) and with said plurality of valves (310a, 310b, 310c, 310d) of said first sealing member (309) in said locked state.

9. The access device (30) according to claim 8, wherein said at least first connection element (317) of said adjustable connecting arrangement comprises at least one recess and said at least second connection element (318) of said adjustable connecting arrangement comprises at least one protrusion.

10. The access device (30) according to claim 8, wherein at least one valve of the plurality of valves (310a, 310b, 310c, 310d) comprises a resilient material that is provided with at least two intersecting slits (311a, 311b) that are arranged to define at least three valve parts (310a-I, 310a-II, 310a-III, 310a-IV) that are adapted for allowing passage of an invasive instrument (10, 20, 240) when said at least three valve parts (310a-I, 310a-II, 310a-III, 310a-IV) are in a first, at least partly opened position and providing a fluid-tight seal when said at least three valve parts (310a-I, 310a-II, 310a-III, 310a-IV) are in a second, closed position.

11. An access device (30) according to claim 1, wherein said outer wall of the shaft (33) further is provided with at least one elongated slot (91a, 91c) that is arranged in an axial direction of said at least one lumen (34a, 34b, 34c, 34d) to provide an increased aperture of said at least one lumen at a distal end part of said at least one lumen.

12. An assembly (1) comprising at least one steerable invasive instrument (10, 20, 240) and an access device (30) according to any one of the preceding claims, the at least one steerable invasive instrument comprising an elongated tubular body (18) having a proximal end part (11, 21) comprising a proximal zone (158) and at least one proximal flexible zone (156) located distal to said proximal zone (158), a distal end part (13, 23) comprising at least one distal flexible zone (154) and a rigid intermediate part (155), wherein the steerable invasive instrument further comprises a steering arrangement that is adapted for translating a deflection of the at least one proximal flexible zone (156) relative to the rigid intermediate part (155) into a related deflection of said at least one distal flexible zone (154).

## Patentansprüche

1. Zugangsvorrichtung (30), die für das Führen mindestens eines lenkbaren invasiven Instrumentes (10, 20) in ein Objekt hinein und aus diesem heraus über eine Zugangsöffnung des Objektes angepasst ist, wobei das mindestens eine lenkbare invasive Instrument umfasst:
einen länglichen röhrenförmigen Körper (18) mit einem proximalen Endteil (11, 21), umfassend eine proximale Zone (158) und mindestens eine von der proximalen Zone (158) entfernt liegende proximale flexible Zone (156), einem distalen Endteil (13, 23), umfassend mindestens eine distale flexible Zone (154) und einen starren Zwischenteil (155), wobei das lenkbare invasive Instrument ferner eine Lenkanordnung umfasst, die für das Übertragen einer Durchbiegung der mindestens einen proximalen flexiblen Zone (156) relativ zu dem starren Zwischenteil (155) in eine ähnliche Durchbiegung der mindestens einen distalen flexiblen Zone (154) angepasst ist;
wobei die Zugangsvorrichtung (30) umfasst:
einen starren Schaft (33), der sich in einer axialen Richtung erstreckt und mindestens ein Lumen (34a, 34b, 34c, 34d) aufweist, wodurch das mindestens eine Lumen so angeordnet ist, dass es einen Durchgang zwischen einer proximalen Stirnfläche und einer distalen Stirnfläche des Schaftes (33) bereitstellt, und für das Aufnehmen und Führen des mindestens einen lenkbaren invasiven Instrumentes in das Objekt hinein und aus diesem heraus in einer parallel zu der axialen Richtung des starren Schaftes (33) verlaufenden Richtung angepasst ist; und **gekennzeichnet durch**
eine Positionseinstellanordnung und eine Durchbiegungseinstellanordnung, umfassend entweder:
einen Stift (50), der sich in einer parallel zu der axialen Richtung des starren Schaftes (33) verlaufenden Richtung erstreckt, wobei der Stift (50) über einen ersten Arm (51) und eine Halterung (31), die eine Vertiefung (40) zum Herstellen eines gegenseitigen Eingriffes mit dem Stift (50), einen Kanal (36) zur Aufnahme des starren Zwischenteiles (12) und einen Schlitz (37) zum lösbaren Koppeln der proximalen Zone (158) des lenkbaren invasiven Instrumentes umfasst, mit dem starren Schaft (33) verbunden ist, wobei die proximale flexible Zone (156) zwischen dem Kanal (36) und dem Schlitz (37) angeordnet ist, derart, dass, wenn das mindestens eine lenkbare invasive Instrument in das mindestens eine Lumen eingeführt wird und die proximale Zone (158) durch die Halterung (31) gekoppelt wird, eine Position des mindestens einen lenkbaren invasiven Instrumentes relativ zu dem starren Schaft (33) in einer parallel zu der axialen Richtung verlaufenden Richtung eingestellt werden kann, und derart, dass ein Winkel zwischen der mindestens einen proximalen flexiblen Zone (156) und dem starren Zwischenteil (155) eingestellt wird, und somit ein Triangulationswinkel zwischen der mindestens einen distalen flexiblen Zone (154) und dem starren Zwischenteil (155) des mindestens einen lenkbaren invasiven Instrumentes (10, 20) eingestellt wird; oder
einen zweiten Arm (61), der mit dem Schaft (33) verbunden ist, und einen dritten Arm (60), der in Bezug auf den zweiten Arm (61) verschiebbar angeordnet ist, wobei sich der zweite Arm (61) in einer parallel zu der axialen Richtung verlaufenden Richtung erstreckt, der dritte Arm (60) mit einem Eingriffsmittel (62; 315, 316) versehen ist, das für das Eingreifen in die proximale Zone (158) des lenkbaren invasiven Instrumentes angepasst ist, derart, dass, wenn das mindestens eine lenkbare Instrument in das mindestens eine Lumen eingeführt wird und in die proximale Zone (158) durch das Eingriffsmittel (62; 315, 316) eingegriffen wird, eine Position des mindestens einen lenkbaren invasiven Instrumentes eingestellt werden kann relativ zu dem Schaft in einer parallel zu der axialen Richtung verlaufenden Richtung, und derart, dass ein Winkel zwischen der mindestens einen proximalen flexiblen Zone (156) und dem starren Zwischenteil (155) eingestellt wird, und somit ein Triangulationswinkel zwischen der mindestens einen distalen flexiblen Zone (154) und dem starren Zwischenteil (155) des mindestens einen lenkbaren invasiven Instrumentes (10, 20) eingestellt wird.

2. Zugangsvorrichtung (30) nach Anspruch 1,
wobei der Stift (50) und die Halterung (31) über den ersten Arm (51) derart schwenkbar mit dem Schaft (33) verbunden sind, dass eine Drehung des lenkbaren invasiven Instrumentes (10, 20) um eine Drehachse herum, die sich in der axialen Richtung des Schaftes (33) erstreckt, eingestellt werden kann.

3. Zugangsvorrichtung (30) nach Anspruch 1, wobei die Halterung (31) eine Mehrzahl von Aufnahmemitteln (40, 41, 42, 43, 44) zum Herstellen eines gegenseitigen Eingriffes mit dem Stift (50) aufweist.

4. Zugangsvorrichtung (30) nach Anspruch 1, wobei eine Wand des mindestens einen Lumens mit mindestens einer länglichen Vertiefung (80, 81) versehen ist, die sich entlang der Länge des mindestens einen Lumens erstreckt.

5. Zugangsvorrichtung (30) nach Anspruch 1, wobei eine Außenwand des Schaftes (33) mit mindestens einer länglichen Vertiefung (90) versehen ist, die sich entlang mindestens eines Teiles der Länge des Schaftes erstreckt.

6. Zugangsvorrichtung (30) nach Anspruch 1, ferner umfassend:
eine Koppelanordnung, die ein erstes Koppelelement (301) und ein zweites Koppelelement (302) umfasst, wobei das erste Koppelelement (301) an einem proximalen Ende des Schaftes (33) angeordnet ist;
ein Deckelement (303), das das zweite Koppelelement (302) der Koppelanordnung umfasst, wobei das zweite Koppelelement (302) mit dem ersten Koppelelement (301) verbindbar ist, um das Deckelement (303) mit dem proximalen Ende des Schaftes (33) zu verbinden, das Deckelement (303) mit einer ersten Mehrzahl von Durchgangslöchern (304a, 304b, 304c, 304d) versehen ist, die zumindest ein erstes Durchgangsloch umfasst, das so angeordnet ist, dass es einen Durchgang zwischen einer proximalen Stirnfläche und einer distalen Stirnfläche des Deckelementes (303) bereitstellt, wobei das zumindest erste Durchgangsloch so angeordnet ist, dass es das Führen des mindestens einen invasiven Instrumentes (10, 20, 240) in das mindestens eine Lumen (34a, 34b, 34c, 34d) hinein und aus diesem heraus ermöglicht.

7. Zugangsvorrichtung nach Anspruch 6, ferner umfassend zumindest eine erste Dichtungsanordnung (306), die zwischen dem Deckelement (303) und der proximalen Stirnfläche des Schaftes (33) angeordnet ist und für das fluiddichte Abdichten des mindestens einen Lumens (34a, 34b, 34c, 34d) des Schaftes (33) angepasst ist.

8. Zugangsvorrichtung nach Anspruch 7, wobei die erste Dichtungsanordnung (306) eine verstellbare Verbindungsanordnung umfasst; und
ein erstes Dichtungsglied (309), das ein elastisches Material umfasst, das mit einer Mehrzahl von Ventilen (310a, 310b, 310c, 310d) und mit zumindest einem ersten Verbindungselement (317) der verstellbaren Verbindungsanordnung versehen ist;
ein zweites Dichtungsglied (313), umfassend ein starres Material, das mit einer zweiten Mehrzahl von Durchgangslöchern (314a, 314b, 314c, 314d) und mit zumindest einem zweiten Verbindungselement (318) der verstellbaren Verbindungsanordnung versehen ist, wobei das zumindest erste Verbindungselement (317) und das zumindest zweite Verbindungselement (318) so ausgestaltet sind, dass sie in einem verriegelten Zustand eine verstellbare Verbindung des ersten Dichtungsgliedes (309) und des zweiten Dichtungsgliedes (313) miteinander ermöglichen, wobei in dem verriegelten Zustand die Mehrzahl von Ventilen (310a, 310b, 310c, 310d) in einer Flucht mit der zweiten Mehrzahl von Durchgangslöchern (314a, 314b, 314c, 314d) angeordnet ist; und
ein drittes Dichtungsglied (307), das mit dem zweiten Dichtungsglied (313) verbunden ist, wobei das dritte Dichtungsglied (307) ein elastisches Material umfasst, das mit einer dritten Mehrzahl von Durchgangslöchern (308a, 308b, 308c, 308d) versehen ist, die in dem verriegelten Zustand in einer Flucht mit der zweiten Mehrzahl von Durchgangslöchern (314a, 314b, 314c, 314d) des zweiten Dichtungsgliedes (313) und mit der Mehrzahl von Ventilen (310a, 310b, 310c, 310d) des ersten Dichtungsgliedes (309) angeordnet ist.

9. Zugangsvorrichtung (30) nach Anspruch 8, wobei das zumindest erste Verbindungselement (317) der verstellbaren Verbindungsanordnung mindestens eine Vertiefung umfasst und das zumindest zweite Verbindungselement (318) der verstellbaren Verbindungsanordnung mindestens einen Vorsprung umfasst.

10. Zugangsvorrichtung (30) nach Anspruch 8, wobei mindestens ein Ventil der Mehrzahl von Ventilen (310a, 310b, 310c, 310d) ein elastisches Material umfasst, das mit mindestens zwei sich überschneidenden Schlitzen (311a, 311b) versehen ist, die so angeordnet sind, dass sie mindestens drei Ventilteile (310a-I, 310a-II, 310a-III, 310a-IV) bestimmen, die angepasst sind für das Ermöglichen des Durchgehens eines invasiven Instrumentes (10, 20, 240), wenn sich die mindestens drei Ventilteile (310a-I, 310a-II, 310a-III, 310a-IV) in einer ersten, zumindest teilweise geöffneten Position befinden, und das Bereitstellen einer fluiddichten Dichtung, wenn sich die mindestens drei Ventilteile (310a-I, 310a-II, 310a-III, 310a-IV) in einer zweiten, geschlossenen Position befinden.

11. Zugangsvorrichtung (30) nach Anspruch 1, wobei die Außenwand des Schaftes (33) ferner mit mindestens einem länglichen Schlitz (91a, 91c) versehen ist, der in einer axialen Richtung des mindestens einen Lumens (34a, 34b, 34c, 34d) angeordnet ist, um eine vergrößerte Öffnung des mindestens einen Lumens an einem distalen Endteil des mindestens einen Lumens bereitzustellen.

12. Anordnung (1), umfassend mindestens ein lenkbares invasives Instrument (10, 20, 240) und eine Zugangsvorrichtung (30) nach einem der vorangehenden Ansprüche, wobei das mindestens eine lenkbare invasive Instrument umfasst: einen länglichen röhrenförmigen Körper (18) mit einem proximalen Endteil (11, 21), umfassend eine proximale Zone (158) und mindestens eine von der proximalen Zone (158) entfernt liegende proximale flexible Zone (156), einem distalen Endteil (13, 23), umfassend mindestens eine distale flexible Zone (154) und einen starren Zwischenteil (155), wobei das lenkbare invasive Instrument ferner eine Lenkanordnung umfasst, die für das Übertragen einer Durchbiegung der mindestens einen proximalen flexiblen Zone (156) relativ zu dem starren Zwischenteil (155) in eine ähnliche Durchbiegung der mindestens einen distalen flexiblen Zone (154) angepasst ist.

## Revendications

1. Dispositif d'accès (30) qui est adapté pour guider au moins un instrument invasif dirigeable (10, 20) à l'intérieur et à l'extérieur d'un objet via un orifice d'accès de l'objet, le au moins un instrument invasif dirigeable comprenant :
un corps tubulaire allongé (18) ayant une partie d'extrémité proximale (11, 21) comprenant une zone proximale (158) et au moins une zone flexible proximale (156) positionnée à distance de ladite zone proximale (158), une partie d'extrémité distale (13, 23) comprenant au moins une zone flexible distale (154) et une partie intermédiaire rigide (155), dans lequel l'instrument invasif dirigeable comprend en outre un agencement de direction qui est adapté pour translater une déflexion de la au moins une zone flexible proximale (156) par rapport à la partie intermédiaire rigide (155) en une déflexion relative de ladite au moins une zone flexible distale (154) ;
dans lequel le dispositif d'accès (30) comprend :
un arbre rigide (33) s'étendant dans une direction axiale et ayant au moins une lumière (34a, 34b, 34c, 34d) moyennant quoi la au moins une lumière est agencée pour fournir un passage entre une face d'extrémité proximale et une face d'extrémité distale de l'arbre (33) et est adapté pour recevoir et guider le au moins un instrument invasif dirigeable à l'intérieur et à l'extérieur de l'objet dans une direction parallèle à ladite direction axiale de l'arbre rigide (33) ; et
**caractérisé par** :
un agencement de réglage de position et un agencement de réglage de déflexion comprenant soit :
une broche (50) s'étendant dans une direction parallèle à ladite direction axiale dudit arbre rigide (33), dans lequel ladite broche (50) est raccordée audit arbre rigide (33) via un premier bras (51) et un support (31) comprenant un évidement (40) pour se mettre en prise avec ladite broche (50), un canal (36) pour recevoir ladite partie intermédiaire rigide (12) et une fente (37) pour coupler, de manière amovible, ladite zone proximale (158) dudit instrument invasif dirigeable, dans lequel ladite zone flexible proximale (156) est agencée entre ledit canal (36) et ladite fente (37), de sorte que lorsque ledit au moins un instrument invasif dirigeable est inséré dans ladite au moins une lumière et que ladite zone proximale (158) est couplée par ledit support (31), une position du au moins un instrument invasif dirigeable peut être réglée par rapport audit arbre rigide (33) dans une direction parallèle à ladite direction axiale et de sorte qu'un angle est réglé entre ladite au moins une zone flexible proximale (156) et ladite partie intermédiaire rigide (155), et réglant ainsi un angle de triangulation entre ladite au moins une zone flexible distale (154) et ladite partie intermédiaire rigide (155) du au moins un instrument invasif dirigeable (10, 20) ; ou
un deuxième bras (61) qui est raccordé à l'arbre (33) et un troisième bras (60) qui est agencé de manière coulissante par rapport audit deuxième bras (61), ledit deuxième bras (61) s'étendant dans une direction parallèle à ladite direction axiale, ledit troisième bras (60) étant prévu avec un moyen de mise en prise (62 ; 315, 316) adapté pour mettre en prise ladite zone proximale (158) dudit instrument invasif dirigeable, de sorte que lorsque ledit au moins un instrument dirigeable est inséré dans ladite au moins une lumière et que ladite zone proximale (158) est mise en prise par ledit moyen de mise en prise (62 ; 315, 316), une position du au moins un instrument invasif dirigeable peut être réglée par rapport à l'arbre dans une direction parallèle à ladite direction axiale et de sorte qu'un angle est réglé entre ladite au moins une zone flexible (156) et la partie intermédiaire rigide (155), et réglant ainsi un angle de triangulation entre ladite au moins une zone flexible distale (154) et ladite partie intermédiaire rigide (155) du au moins un instrument invasif dirigeable (10, 20).

2. Dispositif d'accès (30) selon la revendication 1,
ladite broche (50) et le support (31) étant raccordés de manière pivotante à l'arbre (33) via le premier bras (51) de sorte qu'une rotation de l'instrument invasif dirigeable (10, 20) peut être réglée autour d'un axe de rotation s'étendant dans la direction axiale de l'arbre (33).

3. Dispositif d'accès (30) selon la revendication 1, dans lequel ledit support (31) a une pluralité de moyens de réception (40, 41, 42, 43, 44) pour se mettre en prise avec la broche (50).

4. Dispositif d'accès (30) selon la revendication 1, dans lequel une paroi de la au moins une lumière est prévue avec au moins un évidement allongé (80, 81) s'étendant le long de la longueur de la au moins une lumière.

5. Dispositif d'accès (30) selon la revendication 1, dans lequel une paroi externe de l'arbre (33) est prévue avec au moins un évidement (90) s'étendant le long d'au moins une partie de la longueur de l'arbre.

6. Dispositif d'accès (30) selon la revendication 1, comprenant en outre :
un agencement de couplage qui comprend un premier élément de couplage (301) et un second élément de couplage (302), dans lequel ledit premier élément de couplage (301) est agencé à une extrémité proximale de l'arbre (33) ;
un élément de recouvrement (303) qui comprend ledit second élément de couplage (302) dudit agencement de couplage, ledit second élément de couplage (302) pouvant se raccorder avec ledit premier élément de couplage (301) pour raccorder ledit élément de recouvrement (303) avec l'extrémité proximale de l'arbre (33), ledit élément de recouvrement (303) étant prévu avec une première pluralité de trous débouchants (304a, 304b, 304c, 304d) comprenant au moins un premier trou débouchant qui est agencé pour fournir un passage entre une face d'extrémité proximale et une face d'extrémité distale de l'élément de recouvrement (303), ledit au moins un premier trou débouchant étant agencé pour permettre le guidage dudit au moins un instrument invasif (10, 20, 240) à l'intérieur et à l'extérieur de ladite au moins une lumière (34a, 34b, 34c, 34d).

7. Dispositif d'accès selon la revendication 6, comprenant en outre au moins un premier agencement d'étanchéité (306) qui est agencé entre l'élément de recouvrement (303) et la face d'extrémité proximale de l'arbre (33) et est adapté pour sceller ladite au moins une lumière (34a, 34b, 34c, 34d) de l'arbre (33) d'une manière étanche au fluide.

8. Dispositif d'accès selon la revendication 7, ledit premier agencement d'étanchéité (306) comprenant un agencement de raccordement ajustable ; et
un premier élément d'étanchéité (309) qui comprend un matériau résilient qui est prévu avec une pluralité de valves (310a, 310b, 310c, 310d) et avec au moins un premier élément de raccordement (317) dudit agencement de raccordement ajustable ;
un deuxième élément d'étanchéité (313) comprenant un matériau rigide qui est prévu avec une deuxième pluralité de trous débouchants (314a, 314b, 314c, 314d) et avec au moins un second élément de raccordement (318) dudit agencement de raccordement ajustable, ledit au moins un premier élément de raccordement (317) et ledit au moins un second élément de raccordement (318) étant configurés pour permettre un raccordement ajustable dudit premier élément d'étanchéité (309) et dudit deuxième élément d'étanchéité (313) entre eux dans un état bloqué, ladite pluralité de valves (310a, 310b, 310c, 310d) étant agencée en alignement avec ladite deuxième pluralité de trous débouchants (314a, 314b, 314c, 314d) dans ledit état bloqué ; et
un troisième élément d'étanchéité (307) qui est raccordé audit deuxième élément d'étanchéité (313), ledit troisième élément d'étanchéité (307) comprenant un matériau résilient qui est prévu avec une troisième pluralité de trous débouchants (308a, 308b, 308c, 308d) qui sont agencés en alignement avec ladite deuxième pluralité de trous débouchants (314a, 314b, 314c, 314d) dudit deuxième élément d'étanchéité (313) et avec ladite pluralité de valves (310a, 310b, 310c, 310d) dudit premier élément d'étanchéité (309) dans ledit état bloqué.

9. Dispositif d'accès (30) selon la revendication 8, dans lequel ledit au moins un premier élément de raccordement (317) dudit agencement de raccordement ajustable comprend au moins un évidement et ledit au moins un second élément de raccordement (318) dudit agencement de raccordement ajustable comprend au moins une saillie.

10. Dispositif d'accès (30) selon la revendication 8, dans lequel au moins une valve de la pluralité de valves (310a, 310b, 310c, 310d) comprend un matériau résilient qui est prévu avec au moins deux fentes d'intersection (311a, 311b) qui sont agencées pour définir au moins trois parties de valve (310a-I, 310a-II, 310a-III, 310a-IV) qui sont adaptées pour permettre le passage d'un instrument invasif (10, 20, 240) lorsque lesdites au moins trois parties de valve (310a-I, 310a-II, 310a-III, 310a-IV) sont dans une première position au moins partiellement ouverte et fournissant un joint d'étanchéité au fluide lorsque lesdites au moins trois parties de valve (310a-I, 310a-II, 310a-III, 310a-IV) sont dans une seconde position fermée.

11. Dispositif d'accès (30) selon la revendication 1, dans lequel ladite paroi externe de l'arbre (33) est en outre prévue avec au moins une fente allongée (91a, 91c) qui est agencée dans une direction axiale de ladite au moins une lumière (34a, 34b, 34c, 34d) pour fournir une ouverture accrue de ladite au moins une lumière au niveau d'une partie d'extrémité distale de ladite au moins une lumière.

12. Ensemble (1) comprenant au moins un instrument invasif dirigeable (10, 20, 240) et un dispositif d'accès (30) selon l'une quelconque des revendications précédentes, le au moins un instrument invasif dirigeable comprenant un corps tubulaire allongé (18) ayant une partie d'extrémité proximale (11, 21) comprenant une zone proximale (158) et au moins une zone flexible proximale (156) positionnée à distance de ladite zone proximale (158), une partie d'extrémité distale (13, 23) comprenant au moins une zone flexible distale (154) et une partie intermédiaire rigide (155), dans lequel l'instrument invasif dirigeable comprend en outre un agencement de direction qui est adapté pour translater une déflexion de ladite au moins une zone proximale (156) par rapport à la partie intermédiaire rigide (155) en une déflexion relative de ladite au moins une zone flexible distale (154).
